# EUROPEAN PATENT APPLICATION

(11) **EP 2 954 924 A1**
(43) Date of publication of application: **16.12.2015**
(21) Application number: 14749649.1
(22) Date of filing: 05.02.2014
(51) Int. Cl.: A61M 35/00, A61F 13/00

(54) **SHEET AFFIXING JIG AND SHEET AFFIXING METHOD**

(30) Priority: 08.02.2013 JP 2013023557
(71) Applicant: OLYMPUS CORPORATION, Shibuya-ku Tokyo 151-0072 (JP)
(72) Inventor: HONDA, Jun, Tokyo 151-0072 (JP); HAYASHI, Masaki, Tokyo 151-0072 (JP)
(74) Representative: Schicker, Silvia
(86) International application number: PCT/JP2014/052687
(87) International publication number: WO 2014/123160

(57) **Abstract**

A sheet-like substance is pasted onto a sheet supporting portion with the position thereof precisely set. Provided is a sheet pasting jig (1) including a blade portion (3) that is brought into close contact with a pasting surface, which is a surface of a sheet supporting portion onto which a sheet-like substance is pasted, and that is capable of cutting the sheet-like substance between the sheet supporting portion and the blade portion into a piece having an area equivalent to or less than that of the pasting surface; and a positioning portion (4) that positions the blade portion (3) and the sheet supporting portion.

## Description

### {Technical Field}

The present invention relates to a sheet pasting jig and a sheet pasting method.

### {Background Art}

In the related art, there are known instruments for transporting and administering a therapeutic substance, which transport a sheet-like therapeutic substance into a body and paste it onto an affected area (for example, see Patent Literature 1).

With these instruments, a therapeutic substance is mounted on a sheet supporting portion so as to close a plurality of openings provided in the flat sheet supporting portion with the sheet-like therapeutic substance, and thus, by reducing the pressure of liquid supplied to the openings, the therapeutic substance is made to adhere thereto and is transported. Then, by increasing the pressure of liquid supplied to the openings in the vicinity of the affected area, the therapeutic substance is made to peel off from the sheet supporting portion and is pasted onto the affected area.

### {Citation List}

### {Patent Literature}

{PTL 1} Japanese Unexamined Patent Application, Publication No. 2008-173333

### {Summary of Invention}

### {Technical Problem}

However, there has been a problem in that, in order to paste the sheet-like therapeutic substance onto the sheet supporting portion, each time the physician needs to cut the therapeutic substance with scissors and set it in place, which is cumbersome.

The present invention has been conceived in light of the above-described circumstances, and an object thereof is to provide a sheet pasting jig and a sheet pasting method with which it is possible to precisely position and paste a sheet-like substance on a sheet supporting portion.

### {Solution to Problem}

In order to achieve the above-described object, the present invention provides the following solutions.

An aspect of the present invention provides a sheet pasting jig including a blade portion that is brought into close contact with a pasting surface, which is a surface of a sheet supporting portion onto which a sheet-like substance is pasted, and that is capable of cutting the sheet-like substance between the sheet supporting portion and the blade portion into a piece having an area equivalent to or less than that of the pasting surface; and a positioning portion that positions the blade portion and the sheet supporting portion.

With this aspect, because the blade portion and the sheet supporting portion are positioned by the positioning portion when the sheet supporting portion is attached to the sheet pasting jig, the sheet-like substance mounted on the sheet supporting portion can be cut with the blade portion into a piece having an area equivalent to or less than that of the pasting surface of the sheet supporting portion and pasted onto the pasting surface in a position-aligned state. In other words, it is not necessary to handle the sheet-like substance that has been cut for the purpose of pasting, and thus, it is possible to perform cutting and pasting in a simple manner.

In the above-described aspect, the positioning portion may have a butting portion that is made to abut against two or more surfaces that intersect each other and that are surfaces other than the pasting surface of the sheet supporting portion.

By doing so, it is possible to position the blade portion and the sheet supporting portion in a simple manner by making the butting portion abut against two or more surfaces of the sheet supporting portion other than the pasting surface thereof.

In the above-described aspect, the sheet supporting portion may be formed in a flat plate shape, and the butting portion may abut against a back surface and two adjacent side surfaces of the pasting surface of the sheet supporting portion.

Ry doing so, the sheet supporting portion can be positioned with respect to the blade portion in the thickness direction of the sheet-like substance by making the back surface of the pasting surface abut against the butting portion, and the sheet supporting portion can be positioned with respect to the blade portion in the two directions perpendicular to the thickness direction of the sheet-like substance by making the adjacent two side surfaces abut against the butting portion.

In the above-described aspect, the positioning portion may have a fitting portion that is fitted to the sheet supporting portion.

By doing so, the sheet supporting portion can be positioned with respect to the blade portion in a simple manner just by fitting the sheet supporting portion to the fitting portion.

In the above-described aspect, the fitting portion may be formed in a depressed shape or a protruding shape that is fitted to a protrusion or a depression provided in the sheet supporting portion.

By doing so, positioning can be performed in a simple manner by fitting the protrusion provided in the sheet supporting portion to the depressed fitting portion or by fitting the depression provided in the sheet supporting portion to the protruded fitting portion.

In the above-described aspect, the fitting portion may have a fitting depression having a shape complementary to an external shape of the sheet supporting portion so that an outer surface of the sheet supporting portion is fitted thereto.

By doing so, positioning can be performed in a simple manner just by fitting the outer surface of the sheet supporting portion to the fitting portion that has the fitting depression having a complementary shape.

The above-described aspect may have a base portion that has a placement portion on which the sheet supporting portion is placed and to which the blade portion is attached, and the positioning portion may have a base-portion protrusion that is provided in the placement portion and that is fitted to a supporting-portion depression that is formed in a surface on the opposite side of the pasting surface of the sheet supporting portion.

By doing so, positioning can be performed in a simple manner just by placing the sheet supporting portion on the placement portion so that the supporting-portion depression is fitted to the base-side protrusion.

In the above-described aspect, when a blade tip of the blade portion cuts the sheet-like substance, the blade portion and the sheet-like substance may be moved relative to each other in a direction parallel to the blade tip.

By doing so, the sheet-like substance is cut by the blade portion in a pulling manner instead of a pushing manner, and thus, it is possible to smoothly cut even a relatively soft jelly-like substance.

In the above-described aspect, a blade tip of the blade portion may be inclined with respect to a direction in which the blade portion is moved.

By doing so, when the blade portion and the sheet-like substance are moved relative to each other in the direction toward the blade tip of the blade portion, at the blade tip inclined with respect to the direction of the movement, relative movement occurs with respect to the sheet-like substance in the direction parallel to the blade tip, and therefore, it is possible to smoothly cut the sheet-like substance.

The above-described aspect may be provided with a moving mechanism that moves a blade tip of the blade portion in a direction parallel to the blade tip relative to the sheet-like substance.

By doing so, the blade portion is moved in the direction parallel to the blade tip with respect to the sheet-like substance by actuating the moving mechanism, and thus, it is possible to smoothly cut the sheet-like substance.

In the above-described aspect, the blade tip may be formed in a corrugated shape along the direction in which the blade portion is moved.

By doing so, because the state of the blade tip that comes into contact with the sheet-like substance changes when the blade portion is moved in the direction parallel to the blade tip, it is possible to smoothly cut the sheet-like substance.

In the above-described aspect, the blade tip of the blade portion may be formed in a ring shape, and the moving mechanism may bring the blade portion into close contact with the sheet-like substance while rotating the blade portion about a center axis of the blade tip.

By doing so, it is possible to smoothly and gradually cut the sheet-like substance in a pulling manner by bringing the ring-shaped blade tip into close contact with the sheet-like substance while rotating the ring-shaped blade tip about the center axis thereof by actuating the moving mechanism.

In the above-described aspect, the blade tip of the blade portion may be formed in a ring shape, and a sheet removing means for removing the sheet-like substance, which has been punched out and remains inside the blade tip, from the blade tip may be provided.

By doing so, it is possible to prevent the occurrence of a problem where the sheet-like substance inside the ring-shaped blade tip peels off from the sheet supporting portion and is removed together with the blade portion when moving the blade tip away from the sheet supporting portion after cutting the sheet-like substance.

In the above-described aspect, the sheet removing means may be provided with a plate member that is attached to the blade portion in a removable manner and that can entirely cover the surface of the sheet supporting portion.

By doing so, by removing the plate member from the blade portion after cutting the sheet-like substance and by pressing the sheet-like substance toward the pasting surface by using a tool inserted into the interior of the ring-shaped blade portion, it is possible to remove the sheet-like substance from the blade tip.

The above-described aspect may be provided with a pressing portion that is formed of an elastic material that can be elastically deformed between a position at which the elastic material protrudes further outward than the blade tip of the blade portion and a position at which the elastic material is retracted further inward than the blade tip, and the sheet removing means may be configured by forming a contact surface of the pressing portion, which comes into contact with the sheet-like substance on the pasting surface, in a mesh pattern.

By doing so, because the contact area between the pasting surface and the sheet-like substance becomes larger than the contact area between the pressing portion and the sheet-like substance, it is possible to make the sheet-like substance remain on the pasting surface to which the sheet-like substance is adhered with a greater adhesive force.

In the above-described aspect, the blade portion may be secured to the plate member, which can entirely cover the surface of the sheet supporting portion.

By doing so, when performing the task of cutting the sheet-like substance by bringing the blade portion into close contact therewith from vertically above by disposing the sheet-like substance with the pasting surface of the sheet supporting portion facing vertically upward, the portion above the sheet-like substance is covered with the plate member. By doing so, dust and dirt falling from above while performing the task can be prevented from becoming attached to the sheet-like substance.

The above-described aspect may be provided with a pressing portion that is formed of an elastic material that can be elastically deformed between a position at which the elastic material protrudes further outward than the blade tip of the blade portion and a position at which the elastic material is retracted further inward than the blade tip.

By doing so, when the blade portion is gradually brought into close contact with the sheet-like substance, the pressing portion that is disposed at the position in which it protrudes further outward than the blade tip comes into contact with the sheet-like substance and holds it down. Then, when the blade portion is brought into closer contact, the pressing portion formed of the elastic material is elastically deformed and compressed, and the sheet-like substance starts to be cut by the blade tip at the point when the blade tip protrudes further outward than the pressing portion. By doing so, because the sheet-like substance continues to be held down even during cutting, it is possible to stably perform the cutting task.

The above-described aspect may be provided with a restricting member that is disposed at a position at which the restricting member is retracted further inward than the blade tip of the blade portion and that restricts the pressing portion from elastically deforming any further.

By doing so, although the pressing portion is further compressed when the blade portion continues to be pressed against the sheet-like substance, once the pressing portion is compressed by a certain amount, the restricting member restricts the pressing portion from elastically deforming any further, and therefore, the pressing portion is limited from pressing the sheet-like substance with an excessive force, which makes it possible to maintain the integrity of the sheet-like substance even when it is soft.

In addition, the above-described aspect may additionally be provided with the sheet supporting portion that is held by means of the positioning portion in a position-aligned state with respect to the blade portion.

By doing so, it is possible to eliminate the task of positioning the sheet supporting portion with respect to the blade portion.

In the above-described aspect, on a back surface on the opposite side of the pasting surface, the sheet supporting portion may have a marker that indicates a position at which the sheet-like substance is to be cut by the blade portion.

By doing so, even if it is not possible to directly observe the position of the sheet-like substance on the pasting surface when pasting the sheet-like substance placed on the sheet supporting portion onto biological tissue, it is possible to ascertain the position of the sheet-like substance based on the marker on the back surface.

In the above-described aspect, at the pasting surface, the sheet supporting portion may have a fold-back portion that protrudes from the pasting surface in the vicinity of the position at which the sheet-like member is to be cut by the blade portion and that has a hook shape that is bent inward of a region to be cut by the blade portion.

By doing so, by means of the fold-back portion being hooked on the edge portion of the sheet-like substance that has been cut by the blade portion, it is possible to prevent the sheet-like substance from falling off the pasting surface.

The above-described aspect may be provided with a cutting-dimension adjusting mechanism that can change a dimension of the sheet-like substance to be cut by the blade portion.

The above-described aspect may be provided with a rotating mechanism that supports the blade portion in a rotatable manner about a predetermined rotation axis, and the cutting-dimension adjusting mechanism may change a distance between the blade portion and the predetermined rotation axis or an angle of the blade portion with respect to the predetermined rotation axis.

By doing so, it is possible to change the diameter of the sheet-like substance to be cut into a circular shape by the blade tip by changing the distance between the blade tip and the rotation axis.

In the above-described aspect, the cutting-dimension adjusting mechanism may be provided with a plurality of the blade portions that have blade tips whose dimensions differ from each other and that are exchangeable.

By doing so, it is possible to change the dimension of the sheet-like substance to be cut by the blade tip just by exchanging the blade portion to be used.

The above-described aspect may be provided with a blade-portion stopping mechanism that stops the movement of the blade portion in the direction in which the blade portion approaches the pasting surface at a position at which the blade portion comes into contact with the pasting surface. Alternatively, the above-described aspect may be provided with a contact-pressure limiting mechanism that limits a contact pressure of the blade portion against the pasting surface within a range that is greater than a pressure required to cut the sheet-like member and that is lower than a pressure that causes the pasting surface to deform.

By doing so, it is possible to prevent the blade tip from wedging into the pasting surface.

The above-described aspect may be provided with a liquid applying mechanism that applies liquid to the pasting surface.

By doing so, by applying the liquid to the sheet-like substance on the pasting surface by means of the liquid applying mechanism, the adhesive force between the sheet-like substance and the affected area is enhanced, and thus, it is possible to facilitate the task of pasting the sheet-like substance onto the affected area.

In addition, another aspect of the present invention provides a pasting method for a sheet-like substance employing any one of sheet pasting jigs described above, including a disposing step of disposing the sheet-like substance on the surface of the sheet supporting portion; a positioning step of positioning the sheet supporting portion and the sheet pasting jig; and a cutting step of cutting the sheet-like substance by means of the blade portion of the sheet pasting jig.

In the above-described aspect, the cutting step may be performed while covering above the sheet-like substance disposed on the surface of the sheet supporting portion.

In addition, in the above-described aspect, the cutting step may be performed while holding down the sheet-like substance, which is disposed on the surface of the sheet supporting portion, against the sheet supporting portion, before cutting and also during cutting.

In addition, in the above-described aspect, the cutting step may be performed while moving a blade tip of the blade portion and the sheet-like substance relative to each other in a direction parallel to the blade tip.

### {Advantageous Effects of Invention}

With the present invention, there is an advantage in that it is possible to precisely position and paste a sheet-like substance onto a sheet supporting portion.

### {Brief Description of Drawings}

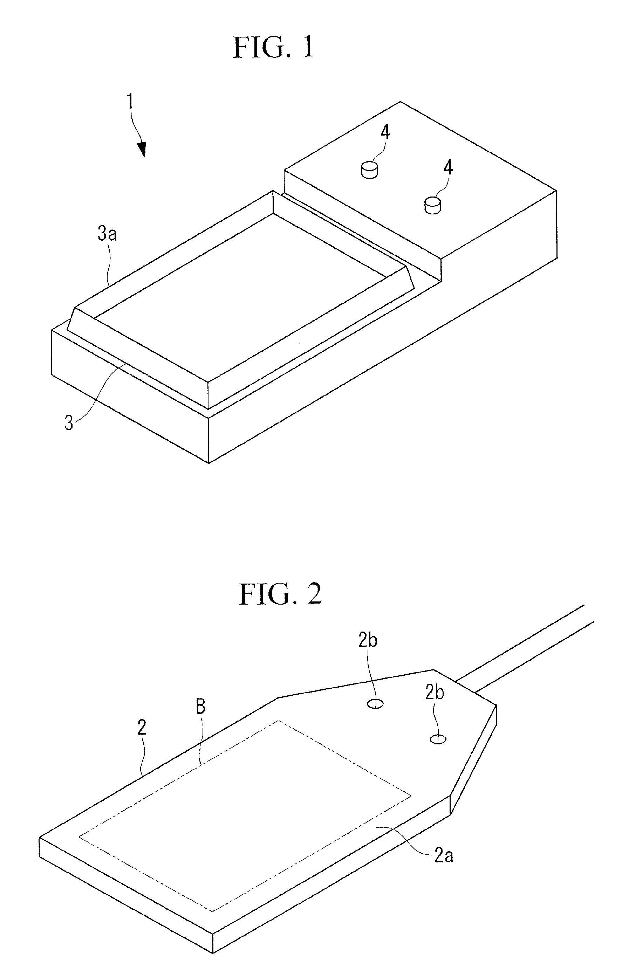

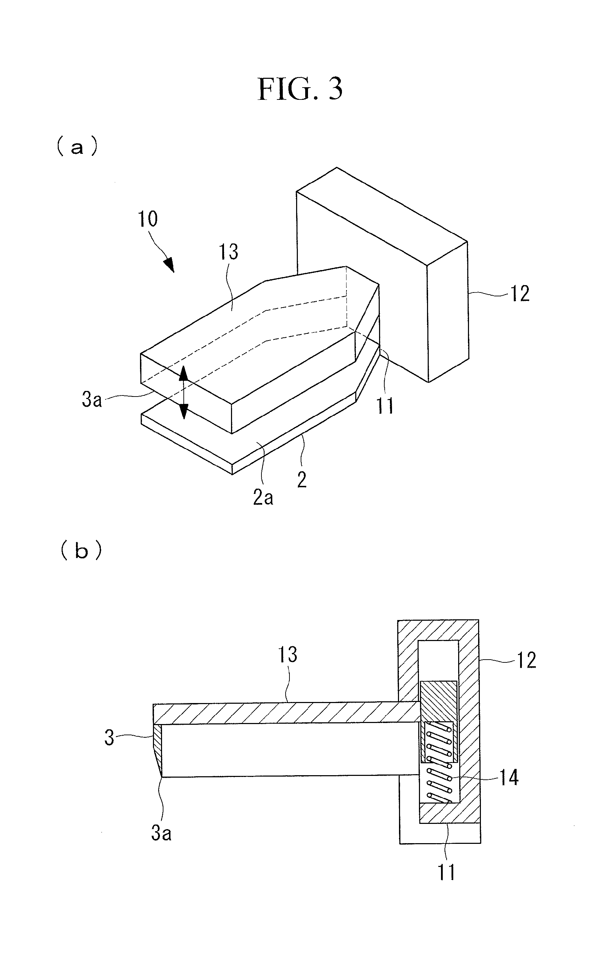

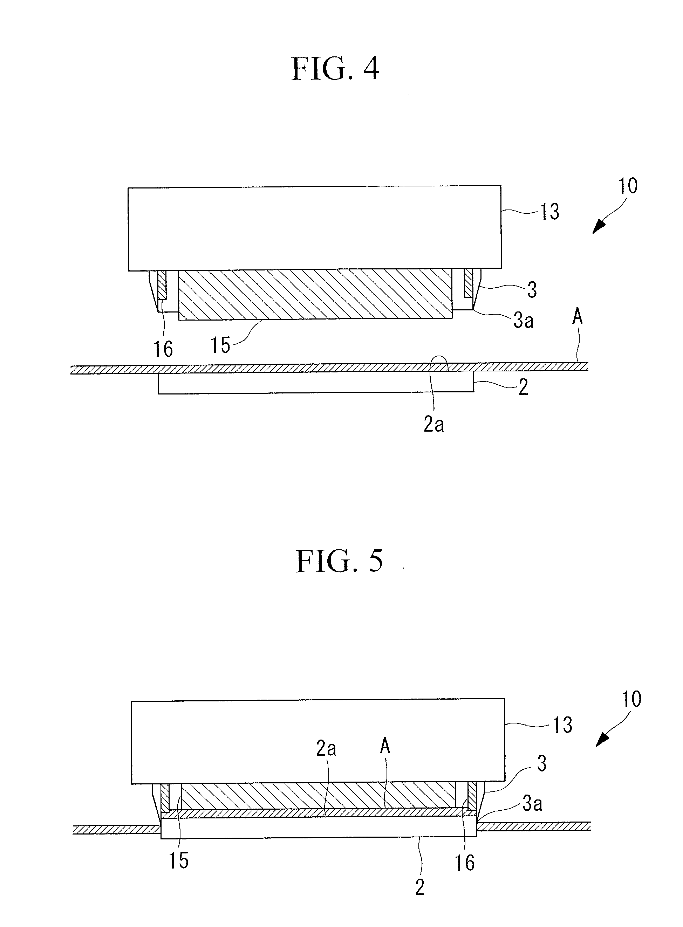

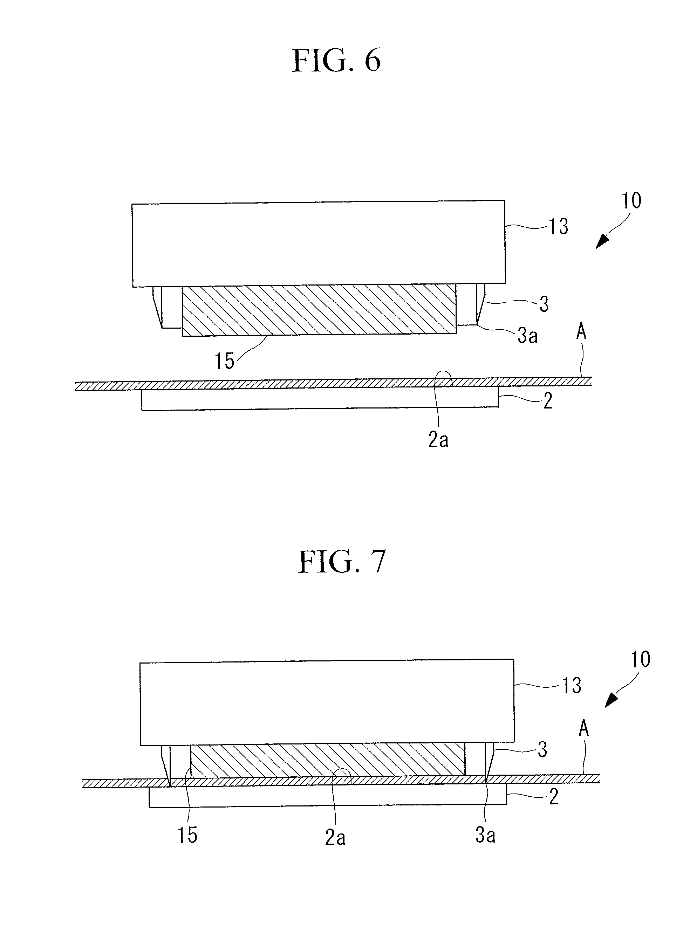

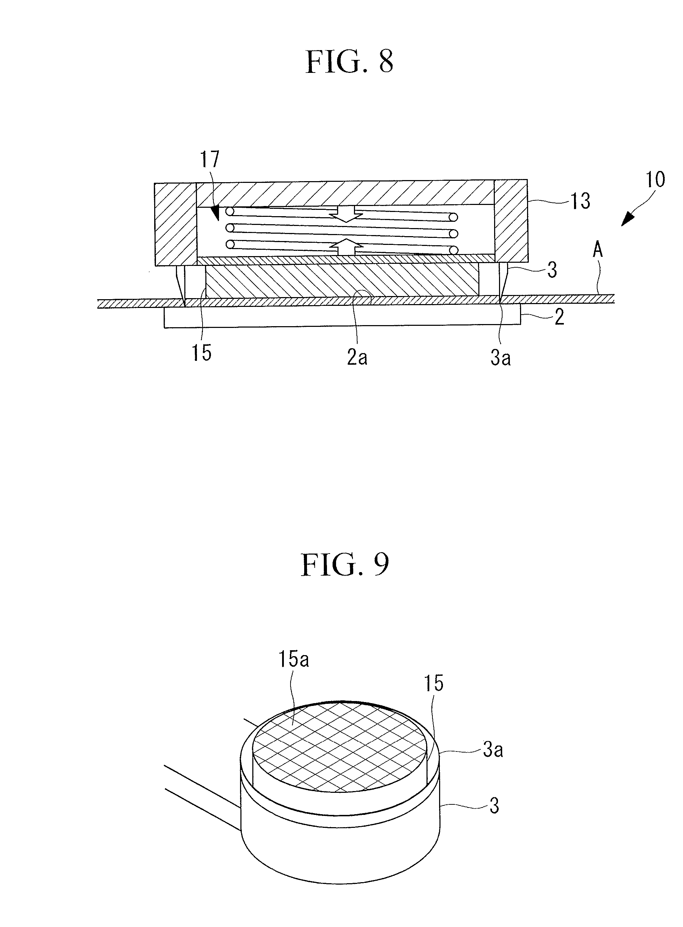

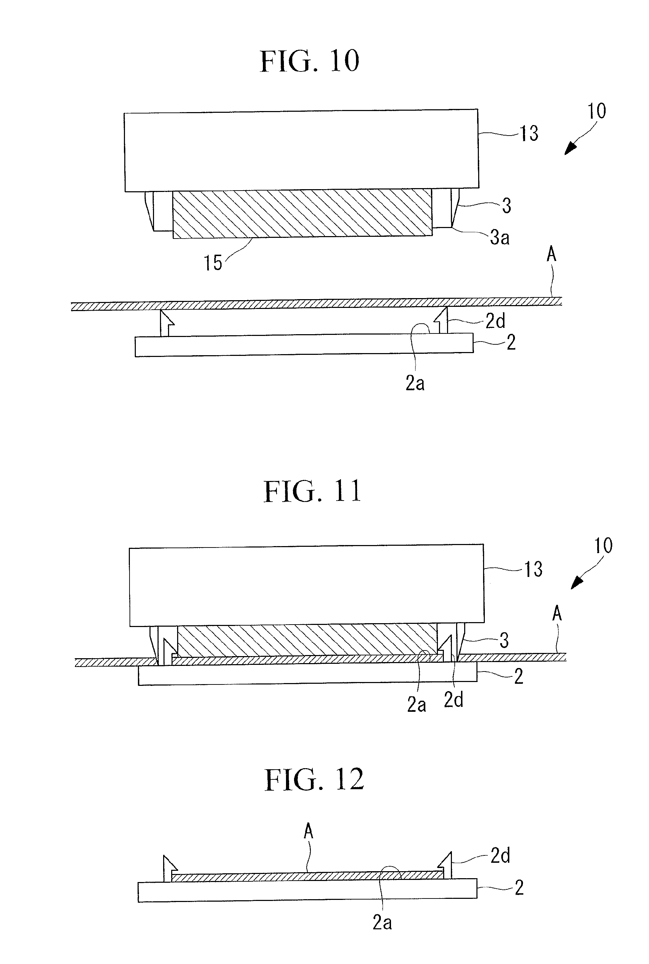

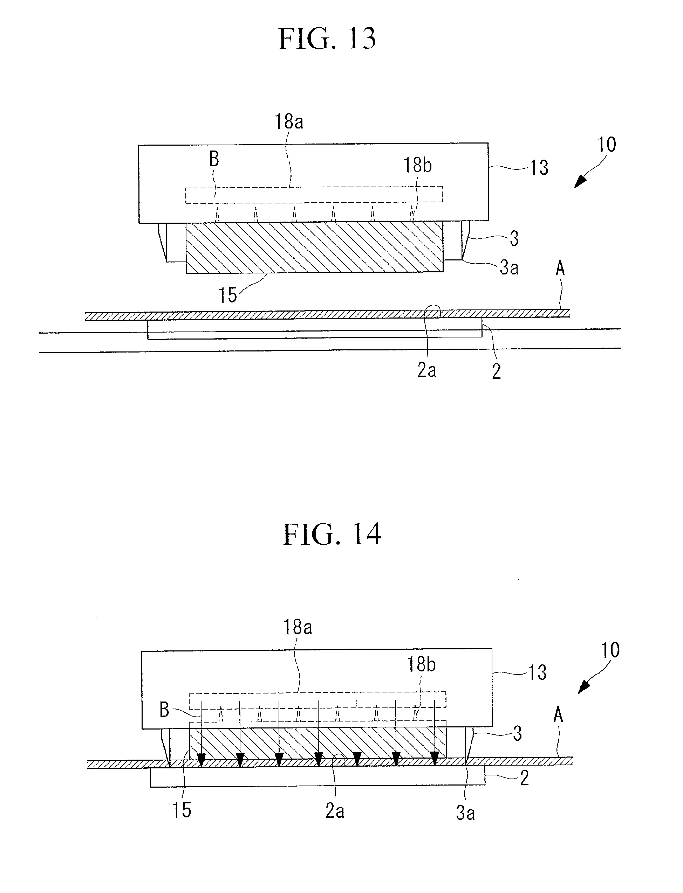

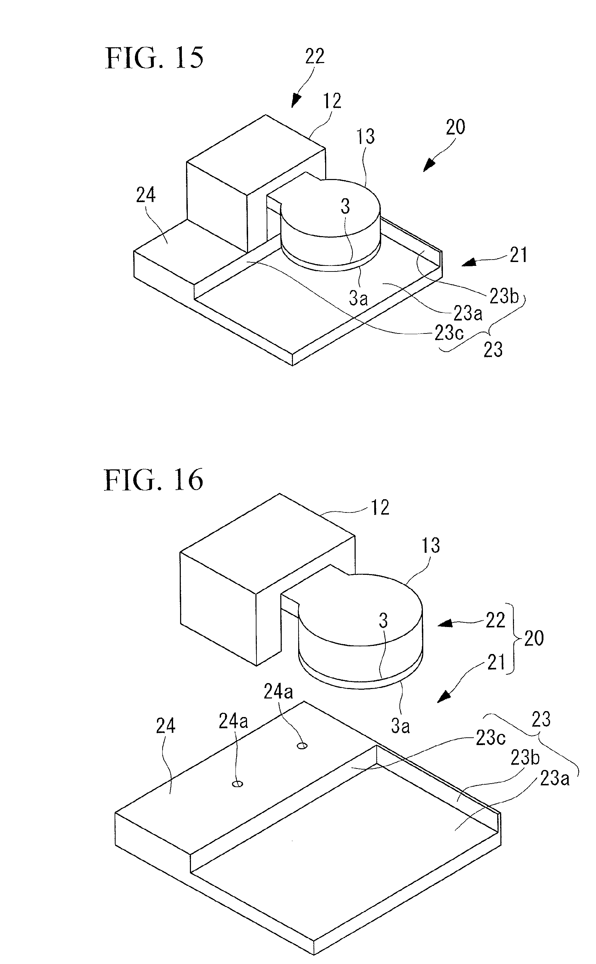

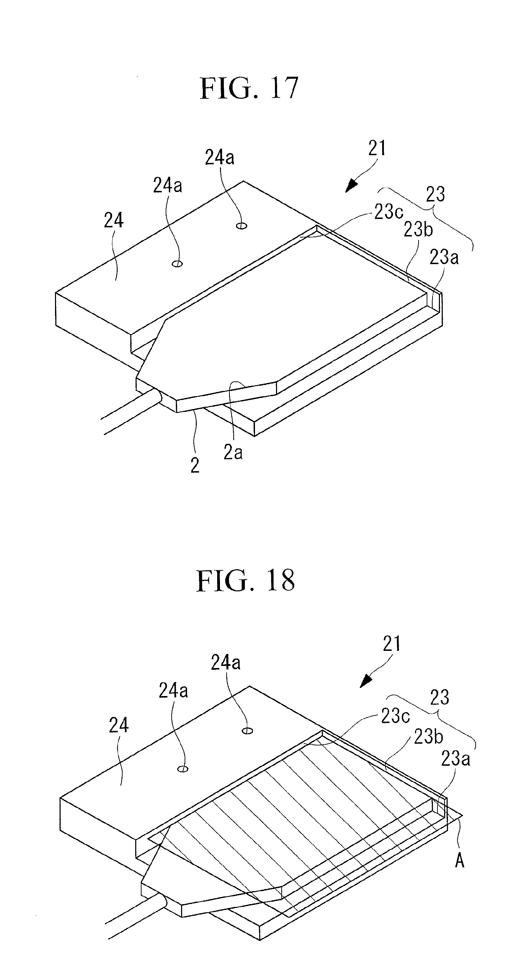

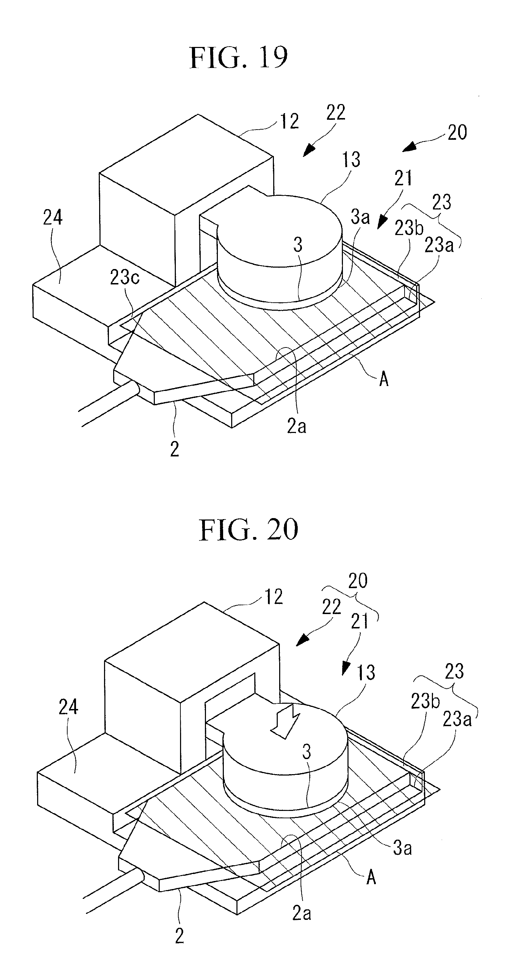

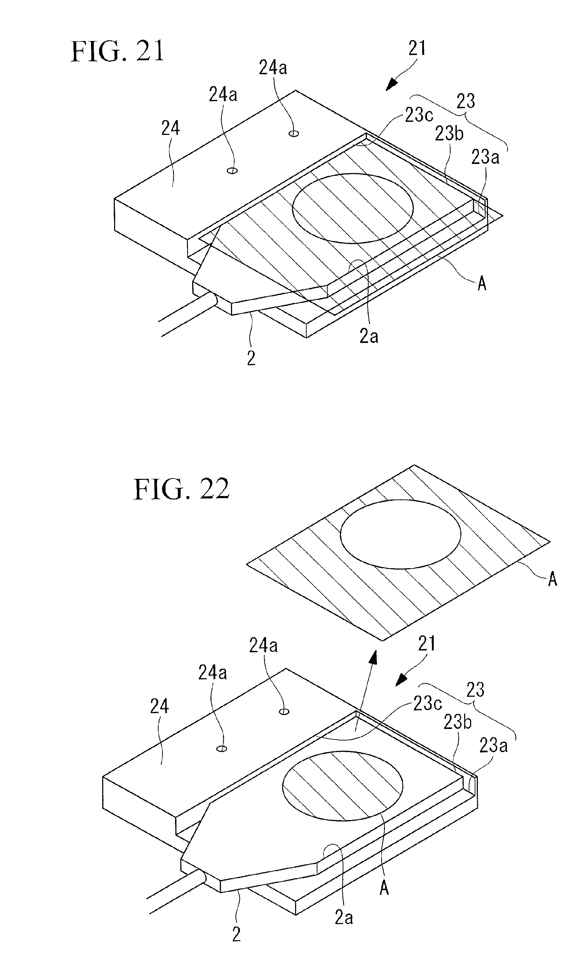

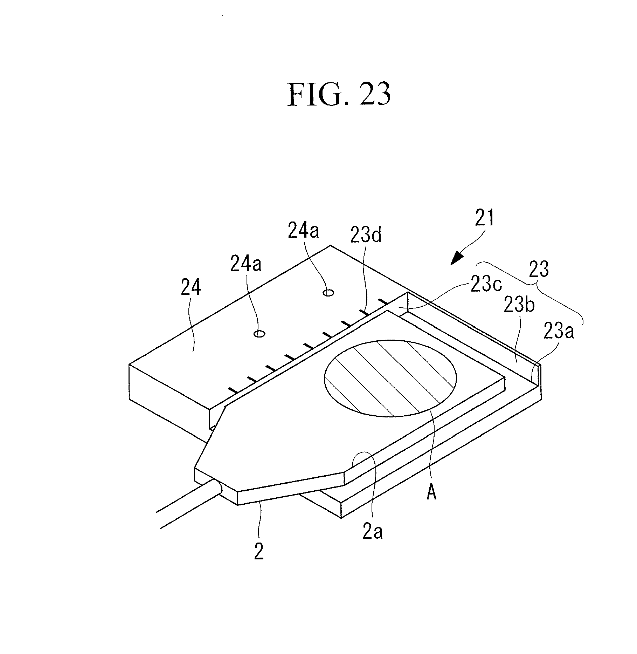

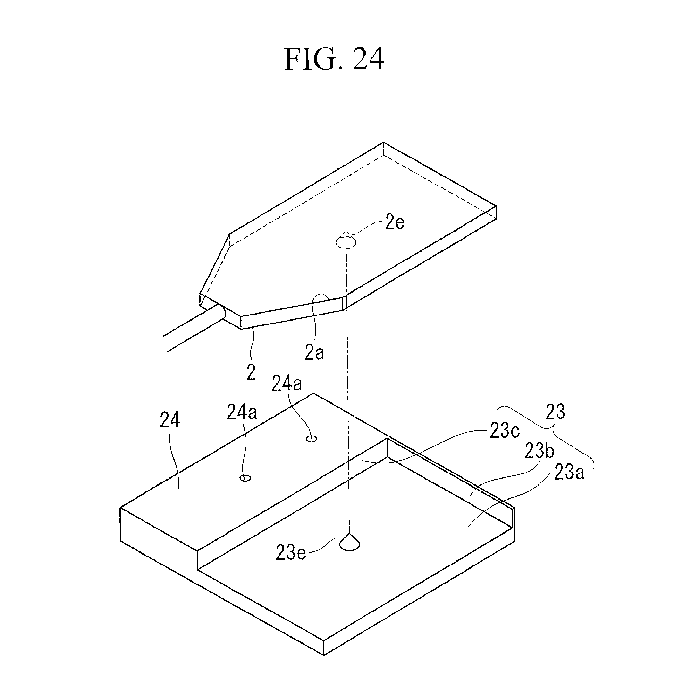

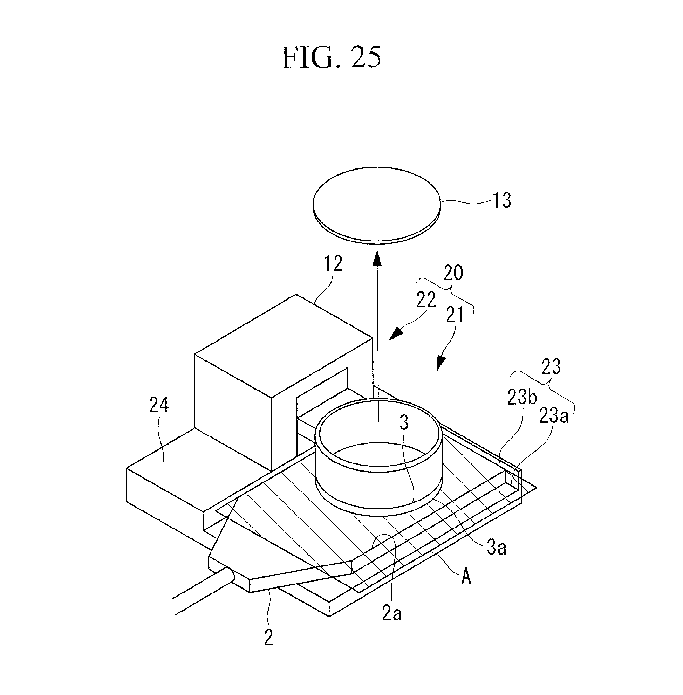

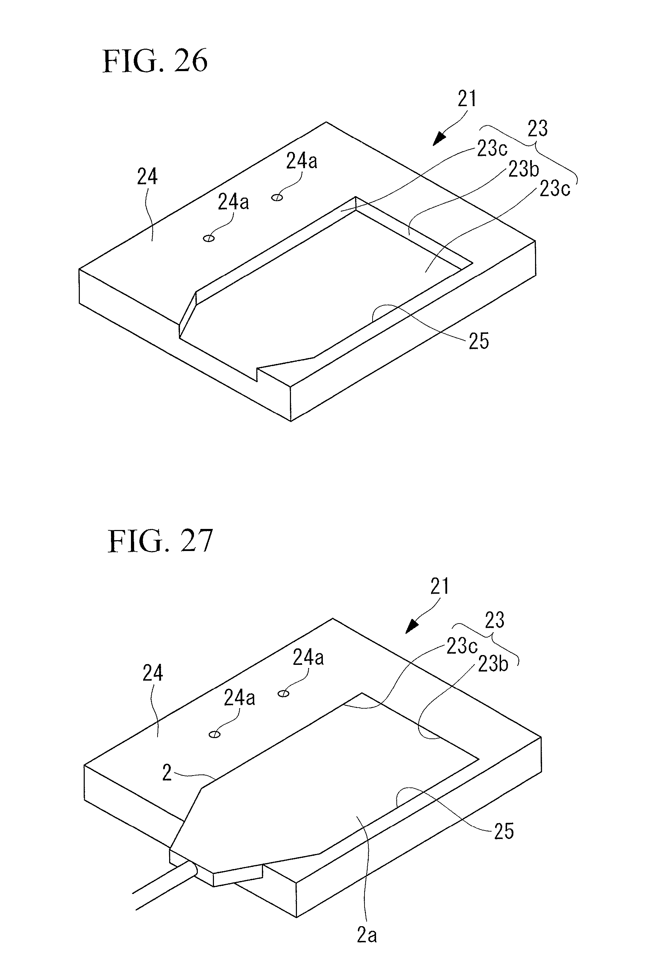

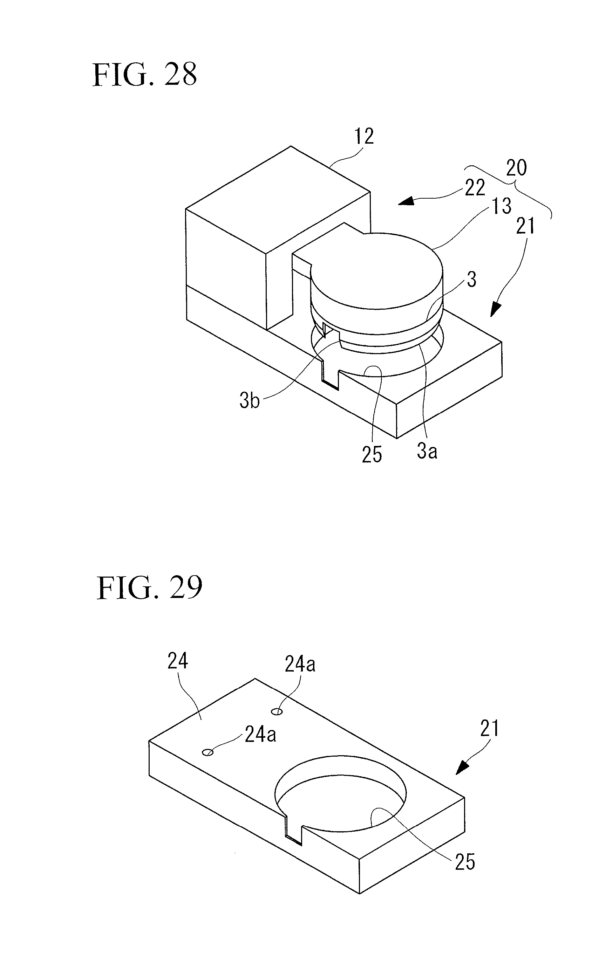

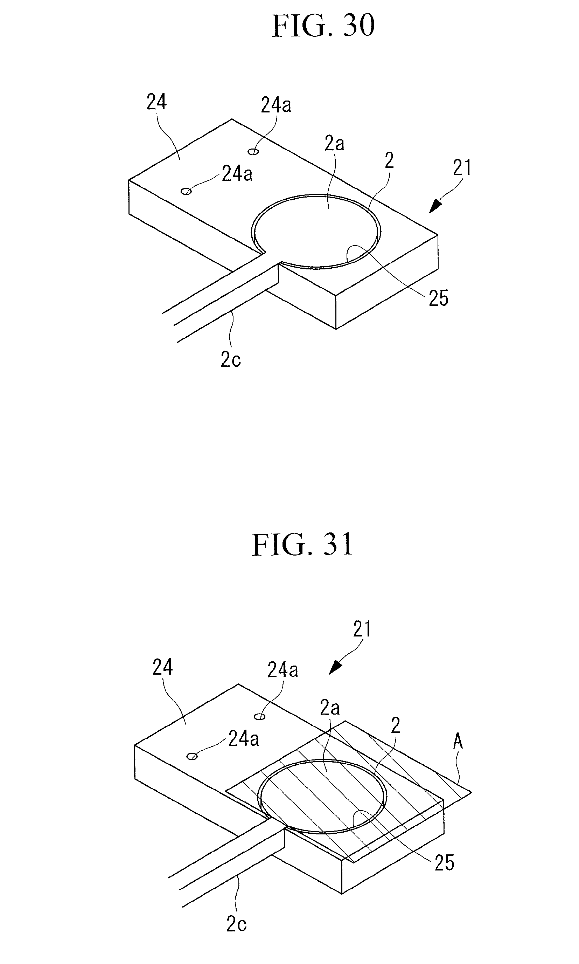

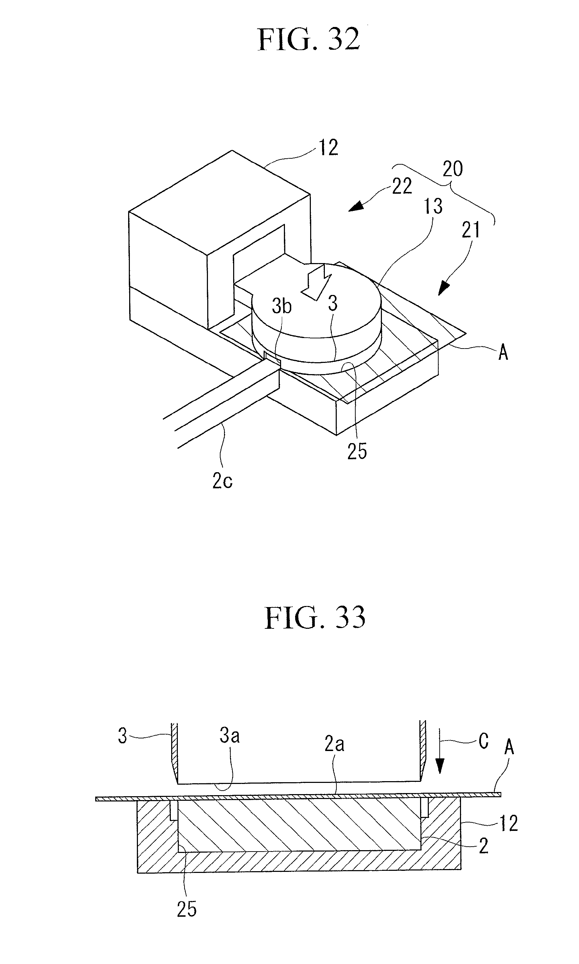

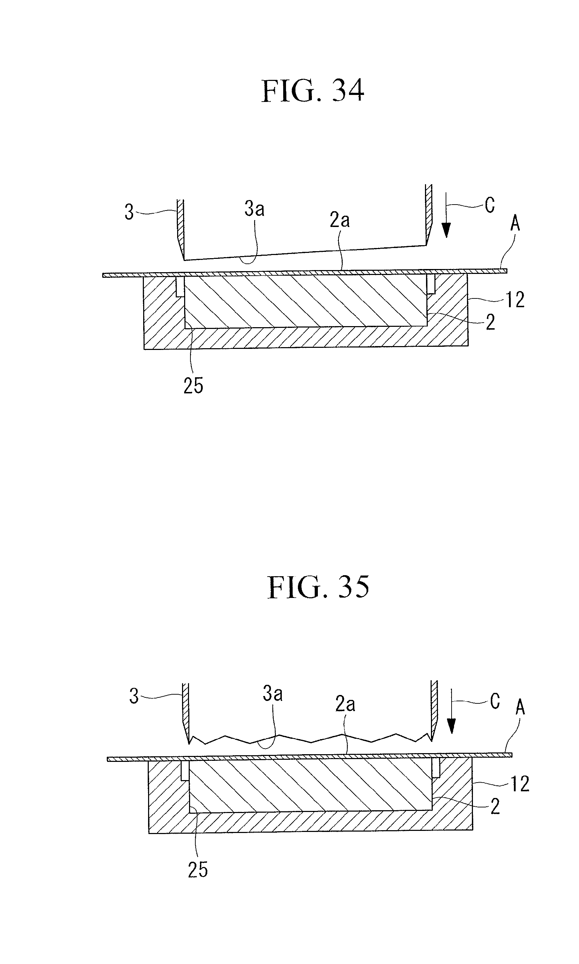

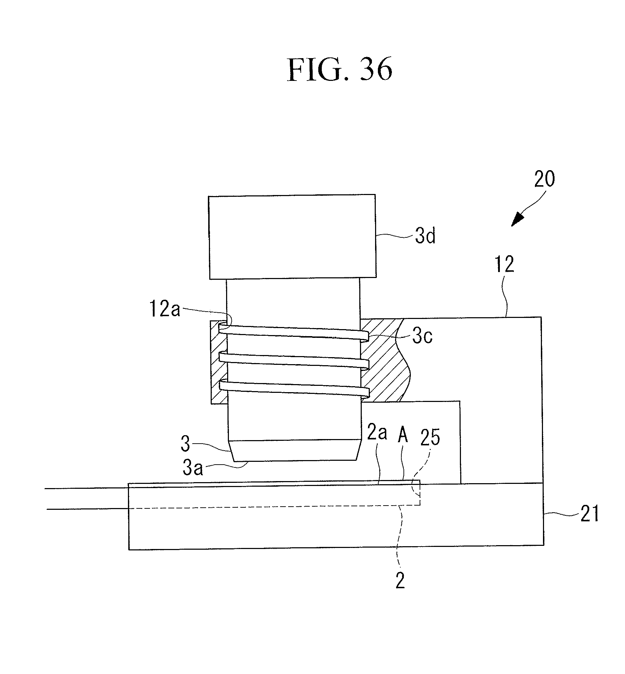

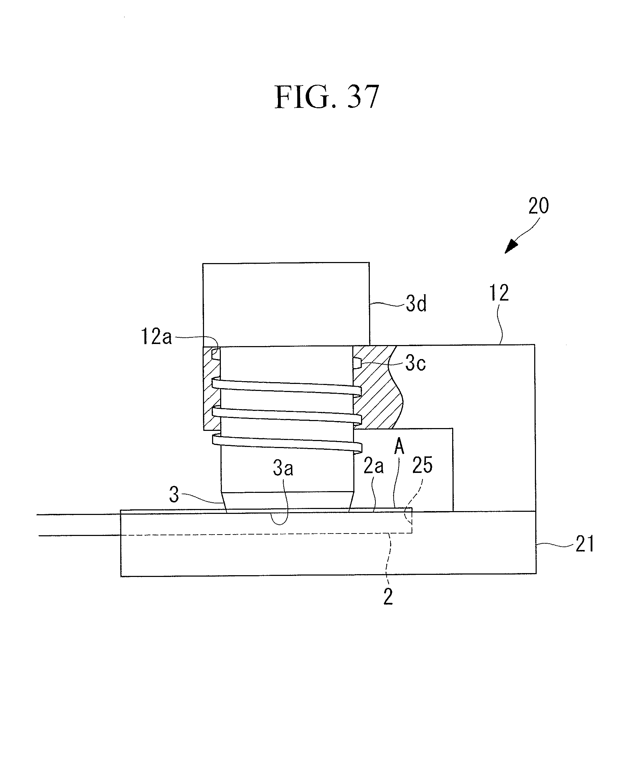

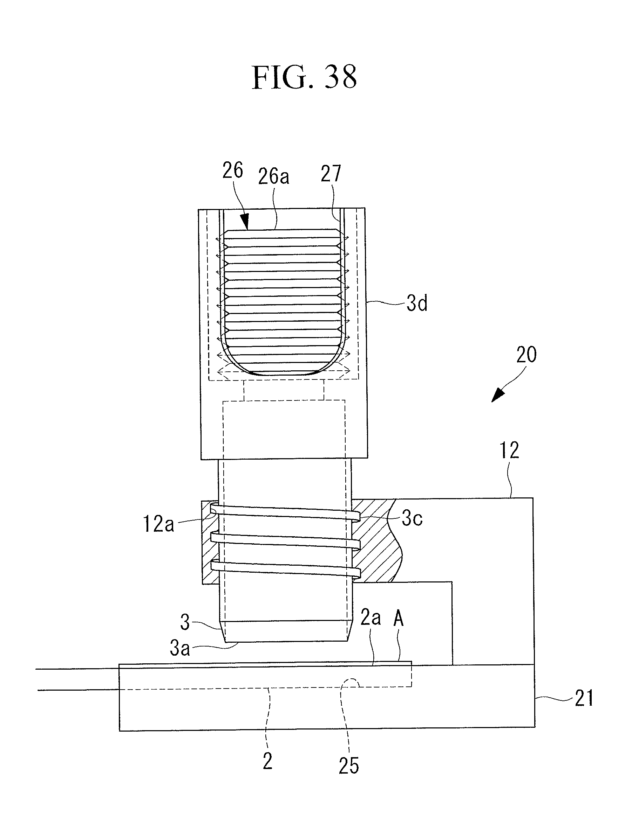

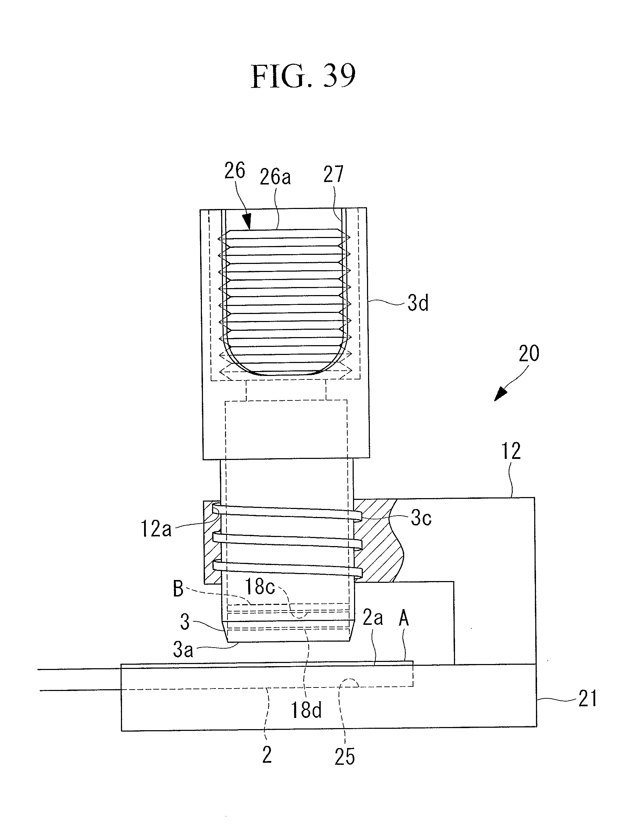

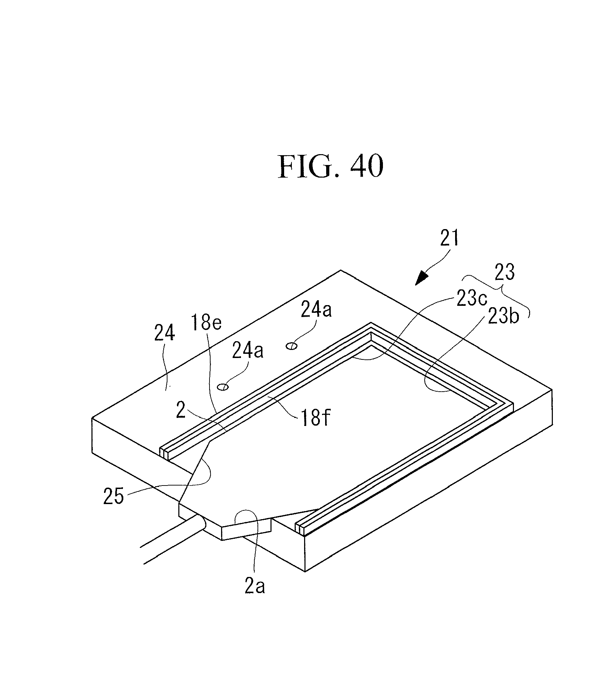

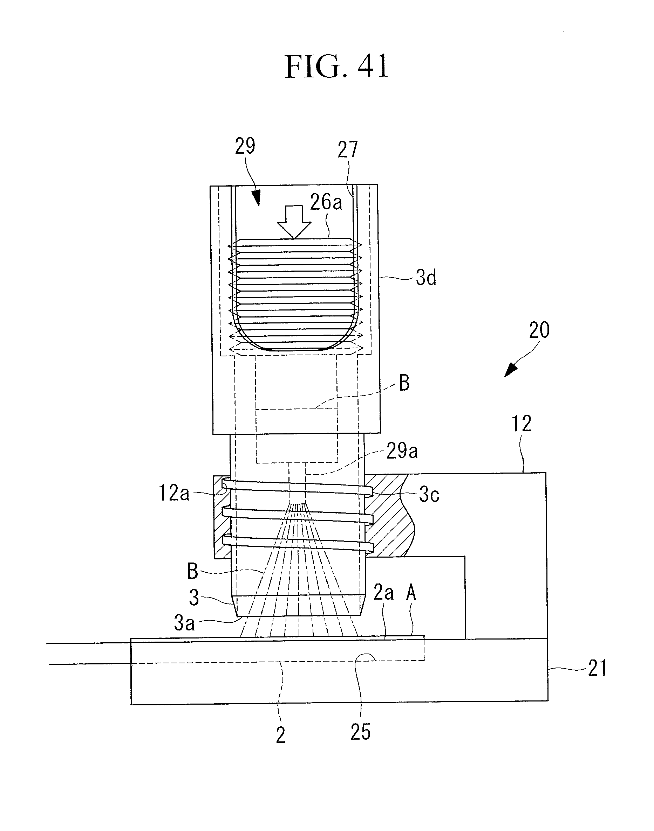

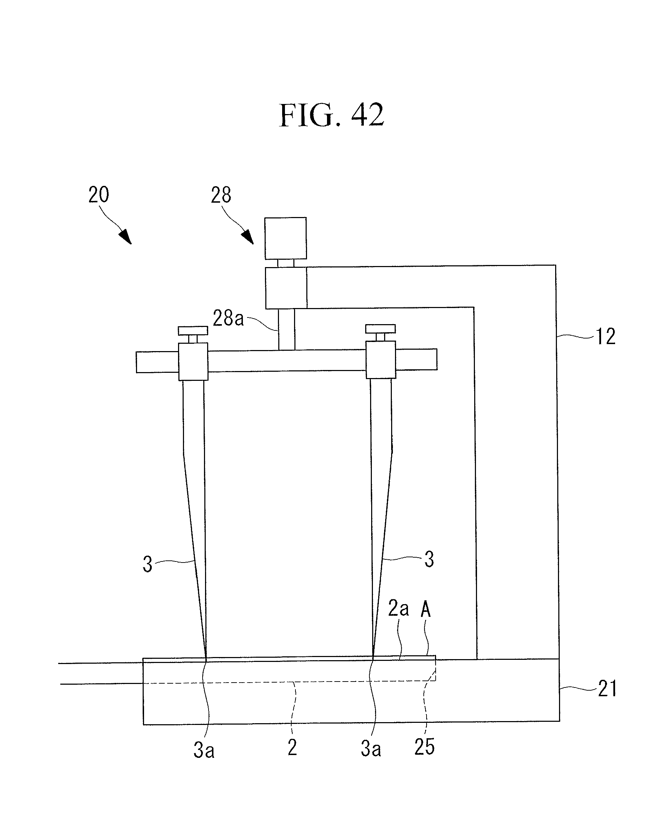

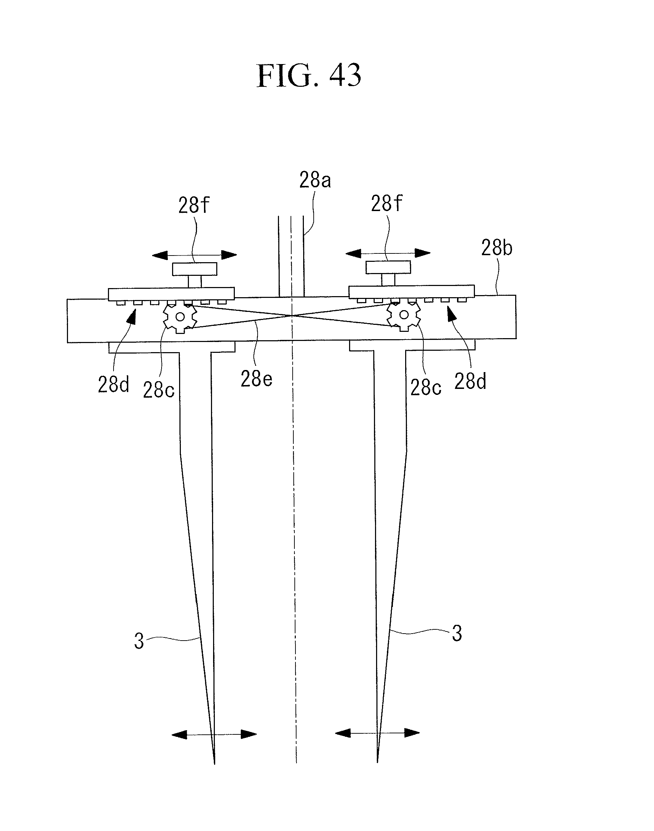

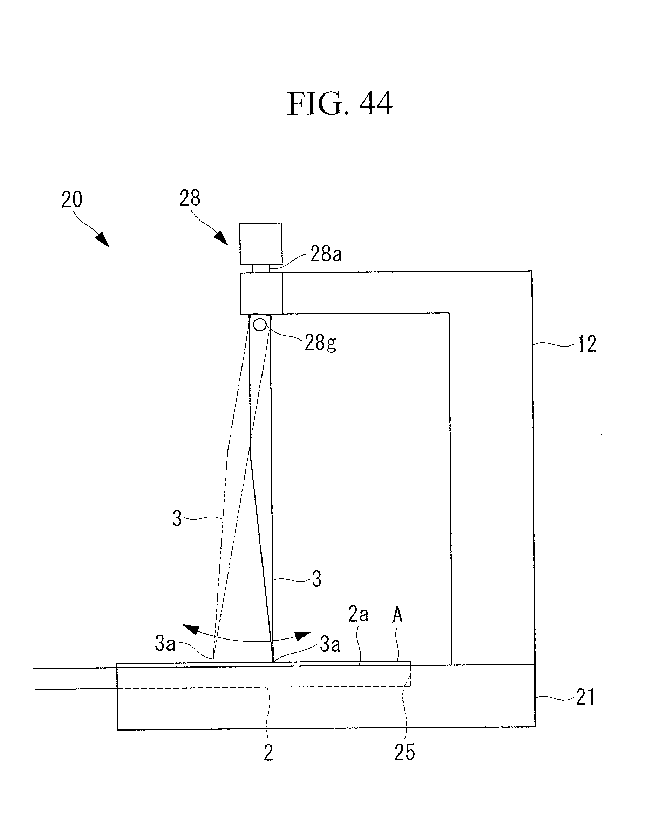

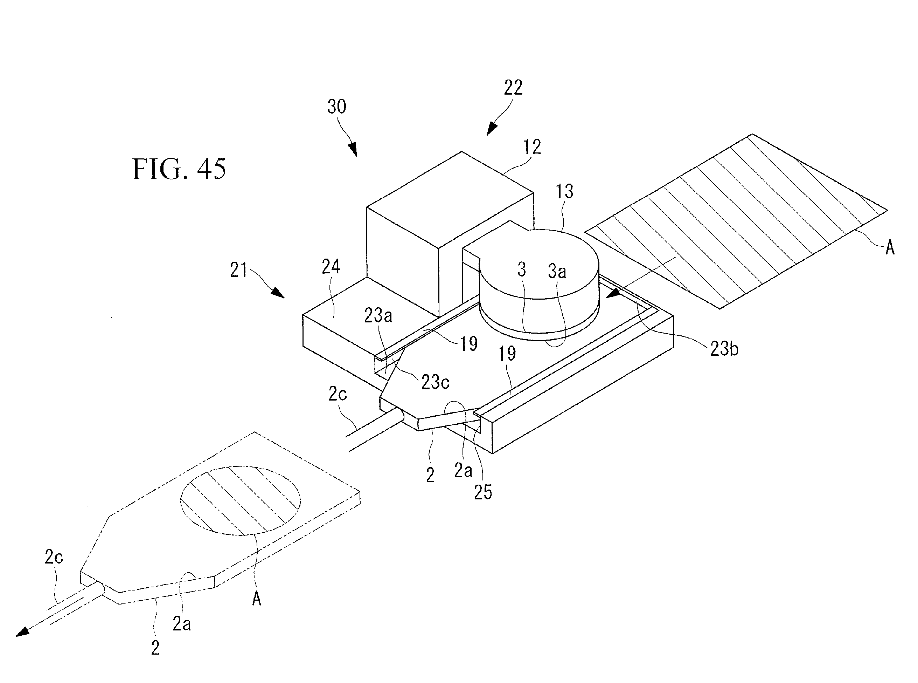

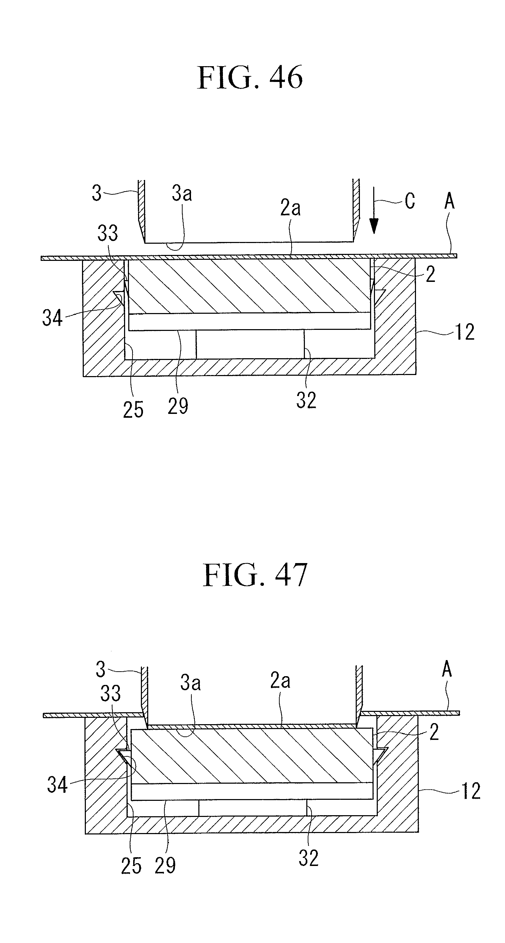

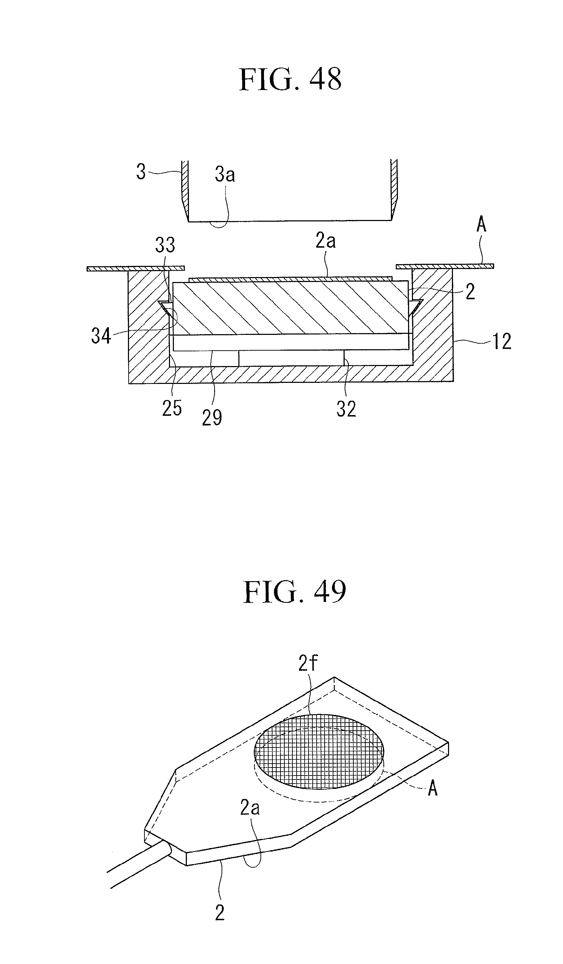

{Fig. 1} Fig. 1 is a perspective view showing a sheet pasting jig according to a first embodiment of the present invention.
{Fig. 2} Fig. 2 is a perspective view showing a sheet supporting portion with which it is possible to attach the sheet pasting jig in Fig. 1 in a state in which the position thereof is set.
{Fig. 3} Fig. 3 shows (a) a perspective view and (b) a longitudinal sectional view showing a sheet pasting jig according to a second embodiment of the present invention.
{Fig. 4} Fig. 4 is a longitudinal sectional view showing a modification of the sheet pasting jig in Fig. 3 in a state before the sheet-like substance is cut.
{Fig. 5} Fig. 5 is a longitudinal sectional view showing a state after the sheet-like substance is cut by the sheet pasting jig in Fig. 4.
{Fig. 6} Fig. 6 is a longitudinal sectional view showing another modification of the sheet pasting jig in Fig. 3 in a state before the sheet-like substance is cut.
{Fig. 7} Fig. 7 is a longitudinal sectional view showing another modification of the sheet pasting jig in Fig. 3.
{Fig. 8} Fig. 8 is a longitudinal sectional view showing another modification of the sheet pasting jig in Fig. 3.
{Fig. 9} Fig. 9 is a perspective view showing a modification of a pressing portion of the sheet pasting jig in Fig. 3.
{Fig. 10} Fig. 10 is a longitudinal sectional view showing a state in which a sheet-like substance is placed on a sheet supporting portion having a fold-back portion.
{Fig. 11} Fig. 11 is a longitudinal sectional view showing a state in which a sheet-like substance has been cut by using the sheet supporting portion in Fig. 10.
{Fig. 12} Fig. 12 is a longitudinal sectional view showing a state in which the sheet supporting portion in Fig. 10 is removed from the sheet pasting jig.
{Fig. 13} Fig. 13 is a longitudinal sectional view showing another modification of the sheet pasting jig in Fig. 3.
{Fig. 14} Fig. 14 is a longitudinal sectional view showing a state in which a sheet-like substance has been cut by using the sheet pasting jig in Fig. 13.
{Fig. 15} Fig. 15 is a perspective view showing a sheet pasting jig according to a third embodiment of the present invention.
{Fig. 16} Fig. 16 is a decomposed perspective view showing the sheet pasting jig in Fig. 15.
{Fig. 17} Fig. 17 is a perspective view showing a state in which the sheet supporting portion is disposed, in a position-aligned state, on a base portion constituting the sheet pasting jig in Fig. 15.
{Fig. 18} Fig. 18 is a perspective view showing a state in which the sheet-like substance is placed on the sheet supporting portion in Fig. 17.
{Fig. 19} Fig. 19 is a perspective view showing a state in which a unit is attached to the base portion in Fig. 18 in a position-aligned state.
{Fig. 20} Fig. 20 is a perspective view for explaining the cutting process of the sheet-like substance performed by using the unit in Fig. 19.
{Fig. 21} Fig. 21 is a perspective view showing a state in which the unit is removed from the base portion after cutting is performed in the state shown in Fig. 20.
{Fig. 22} Fig. 22 is a perspective view showing a state in which a peripheral portion has been removed from the sheet-like substance from the state shown in Fig. 21.
{Fig. 23} Fig. 23 is a perspective view showing a modification of the base portion of the sheet pasting jig in Fig. 15.
{Fig. 24} Fig. 24 is a perspective view showing another modification of the base portion of the sheet pasting jig in Fig. 15.
{Fig. 25} Fig. 25 is a perspective view showing modifications of a blade portion and a plate member of the sheet pasting jig in Fig. 15.
{Fig. 26} Fig. 26 is a perspective view showing another modification of the base portion of the sheet pasting jig in Fig. 15.
{Fig. 27} Fig. 27 is a perspective view showing a state in which the sheet supporting portion is disposed in a state fitted to a fitting depression of the base portion in Fig. 27.
{Fig. 28} Fig. 28 is a perspective view showing a modification of the sheet pasting jig in Fig. 15.
{Fig. 29} Fig. 29 is a perspective view showing a base portion constituting the sheet pasting jig in Fig. 28.
{Fig. 30} Fig. 30 is a perspective view showing a state in which the sheet supporting portion is fitted to the base portion in Fig. 29.
{Fig. 31} Fig. 31 is a perspective view showing a state in which the sheet-like substance is disposed on a pasting surface of the sheet supporting portion in Fig. 30.
{Fig. 32} Fig. 32 is a perspective view for explaining the cutting process performed by using the unit attached to the base portion in Fig. 31 in a position-aligned state.
{Fig. 33} Fig. 33 is a longitudinal sectional view showing a state before the sheet-like substance is cut by using the sheet pasting jig in Fig. 26.
{Fig. 34} Fig. 34 is a longitudinal sectional view showing a first modification of the blade-tip shape of the sheet pasting jig in Fig. 33.
{Fig. 35} Fig. 35 is a longitudinal sectional view showing a second modification of the blade-tip shape of the sheet pasting jig in Fig. 33.
{Fig. 36} Fig. 36 is a partially cutaway front view showing another modification of the sheet pasting jig in Fig. 15.
{Fig. 37} Fig. 37 is a partially cutaway front view showing a modification of the sheet pasting jig in Fig. 36.
{Fig. 38} Fig. 38 is a partially cutaway front view of another modification of the sheet pasting jig in Fig. 15.
{Fig. 39} Fig. 39 is a partially cutaway front view showing a modification of the sheet pasting jig in Fig. 38.
{Fig. 40} Fig. 40 is a perspective view showing a modification of a base portion of the sheet pasting jig in Fig. 38.
{Fig. 41} Fig. 41 is a partially cutaway front view showing another modification of the sheet pasting jig in Fig. 38.
{Fig. 42} Fig. 42 is a front view showing another modification of the sheet pasting jig in Fig. 15.
{Fig. 43} Fig. 43 is a partial configuration diagram showing a modification of the sheet pasting jig in Fig. 42.
{Fig. 44} Fig. 44 is a partial configuration diagram showing another modification of the sheet pasting jig in Fig. 42.
{Fig. 45} Fig. 45 is a perspective view showing another modification of the sheet pasting jig in Fig. 15.
{Fig. 46} Fig. 46 is a longitudinal sectional view showing a modification of a base portion of the sheet pasting jig in Fig. 45.
{Fig. 47} Fig. 47 is a longitudinal sectional view showing a state in which a sheet-like substance has been cut by using the sheet pasting jig in Fig. 45.
{Fig. 48} Fig. 48 is a longitudinal sectional view showing a state in which a blade portion is raised from the state shown in Fig. 47.
{Fig. 49} Fig. 49 is a perspective view showing another modification of the sheet supporting portion.

### {Description of Embodiments}

### {First Embodiment}

A sheet pasting jig 1 according to a first embodiment of the present invention will be described below with reference to the drawings.

The sheet pasting jig 1 according to this embodiment is a jig with which a sheet-like therapeutic substance A is pasted onto a surface (hereinafter, referred to as a pasting surface) 2a of a flat plate-like sheet supporting portion 2 of a pasting device, as shown in Fig. 2, and is provided with a blade portion 3 that is brought into close contact with the pasting surface 2a and that is capable of cutting the sheet-like therapeutic substance A into a piece whose area is equivalent to or less than that of the pasting surface 2a, between the blade portion 3 and the sheet supporting portion 2, and a positioning portion 4 that positions the blade portion 3 and the sheet supporting portion 2.

As shown in Fig. 1, the blade portion 3 is formed into a rectangular shape that is smaller than the pasting surface 2a of the sheet supporting portion 2.

The positioning portions 4 are formed of protrusions (hereinafter, also referred to as the protrusions 4) that are fitted into depressions 2b provided in the pasting surface 2a of the sheet supporting portion 2. The depressions 2b and the protrusions 4 are provided at two locations with the same spacing therebetween.

A surface of the sheet pasting jig 1 on which the protrusions 4 are provided is set at a position nearly equal in height with a blade tip 3a of the blade portion 3. The depressions 2b of the sheet supporting portion 2 are disposed on the same plane as the pasting surface 2a on which the sheet-like therapeutic substance A is mounted. Specifically, when the protrusions 4 of the sheet pasting jig 1 are fitted to the depressions 2b of the sheet supporting portion 2, the blade portion 3 is disposed at a position in which the blade tip 3a comes into contact with the pasting surface 2a of the sheet supporting portion 2 (the position indicated by a chain line B in Fig. 2).

The operation of the thus-configured sheet pasting jig 1 according to this embodiment will now be described.

In order to paste the sheet-like therapeutic substance A onto the pasting surface 2a of the sheet supporting portion 2 of the pasting device by using the sheet pasting jig 1 according to this embodiment, the pasting surface 2a is made to face vertically upward, as shown in Fig. 2, and the therapeutic substance A, which has a large enough size to cover the entire pasting surface 2a or a large enough size to encompass at least the area indicated by the chain line B in Fig. 2, is placed on the pasting surface 2a.

In this state, the protrusions 4 of the sheet pasting jig 1 according to this embodiment are aligned with and fitted into the depressions 2b of the sheet supporting portion 2. By fitting the protrusions 4 into the depressions 2b, it is possible to attach the sheet pasting jig 1 to the sheet supporting portion 2 with the position thereof set. Then, by proceeding to fit the protrusions 4 into the depressions 2b, the blade tip 3a of the blade portion 3 is brought into close contact with the pasting surface 2a, and, by pressing the blade tip 3a against the pasting surface 2a, it is possible to cut the therapeutic substance A between the blade portion 3 and sheet supporting portion 2 by using the blade portion 3.

Specifically, with the sheet pasting jig 1 according to this embodiment, a sheet-like therapeutic substance A whose size is equal to or less than the area of the pasting surface 2a of the sheet supporting portion 2 can be cut and pasted onto the pasting surface 2a of the sheet supporting portion 2 with the position thereof set. As a result, there is an advantage in that cutting of the sheet-like therapeutic substance A and pasting thereof onto the pasting surface 2a of the sheet supporting portion 2 can be performed in a single step.

### {Second Embodiment}

Next, a sheet pasting jig 10 according to a second embodiment of the present invention will be described below with reference to the drawings.

In describing this embodiment, the same reference signs are assigned to the parts having common configurations with those of the sheet pasting jig 1 according to the first embodiment described above, and descriptions thereof will be omitted.

As shown in Fig. 3, a sheet pasting jig 10 according to this embodiment is provided with a bracket 12 that has a fitting portion (positioning portion) 11 into which a side surface of the sheet supporting portion 2 is fitted, a blade portion 3 that is supported in a movable manner with respect to the bracket 12, a plate member 13 that covers the entire blade portion 3, and a spring 14 that biases the blade portion 3 in the direction toward the blade base.

As shown in Fig. 3 (a), the pasting surface 2a of the sheet supporting portion 2 is made to face vertically upward, and a sheet-like therapeutic substance A that covers the entire pasting surface 2a and that has a larger size than the pasting surface 2a is disposed on the pasting surface 2a. Next, the sheet pasting jig 10 is brought into close contact therewith from vertically above, and the side surface of the sheet supporting portion 2 is fitted into the fitting portion 11 of the bracket 12. By doing so, the sheet pasting jig 10 is attached to the sheet supporting portion 2 with the position thereof set, and the portion above the sheet-like therapeutic substance A is covered with the plate member 13.

In this state, by lowering the plate member 13 and the blade portion 3 against the biasing force of the spring 14, the sheet-like therapeutic substance A can be cut with the blade tip 3a of the blade portion 3 facing downward and pasted over the entire pasting surface 2a of the sheet supporting portion 2.

In the example shown in Fig. 3, the blade portion 3 has the same shape as the external shape of the sheet supporting portion 2, and, by lowering the blade portion 3 outside the outer edge of the sheet supporting portion 2 along the outer edge, the sheet-like therapeutic substance A can be cut in a simple manner by means of shearing.

With the thus-configured sheet pasting jig 10 according to this embodiment, there is an advantage in that, when performing the task of cutting the sheet-like therapeutic substance A by using the blade portion 3, because the portion above the sheet-like therapeutic substance A in the region to be pasted onto the pasting surface 2a is covered with the plate member 13, dust and dirt falling from above while performing the task can be prevented from becoming attached to the portion of the sheet-like therapeutic substance A to be pasted.

Note that, as shown in Figs. 4 and 5, the sheet pasting jig 10 according to this embodiment may be provided with, inside the blade portion 3 of the sheet pasting jig 10, a pressing portion 15 formed of an elastic material that can be elastically deformed between a position in which it protrudes further outward than the blade tip 3a of the blade portion 3 and a position in which it is retracted further inward than the blade tip 3a and a restricting member 16 that is disposed at a position in which it is retracted further inward than the blade tip 3a of the blade portion 3 and that restricts the pressing portion 15 from elastically deforming any further.

The pressing portion 15 is formed of an elastic material such as rubber, sponge, or the like. This pressing portion 15 is configured so that, in the stage prior to the blade portion 3 coming into contact with the sheet-like therapeutic substance A, it is possible to make the pressing portion 15 protrude further outward than the blade tip 3a of the blade portion 3 so as to be closer to the distal end, as shown in Fig. 4, and so that, in the stage after the sheet-like therapeutic substance A has been cut by the blade portion 3, it is possible to make the pressing portion 15 elastically deform to be at a position in which it is retracted further inward than the blade tip 3a so as to be closer to the blade base, as shown in Fig. 5.

In addition, the restricting member 16 is configured so that the restricting member 16 is disposed at a position in which it is retracted further inward than the blade tip 3a of the blade portion 3 when the restricting member 16 is brought into close contact with the blade portion 3 on the inside thereof, and so that, as the pressing portion 15 is gradually compressed, the restricting member 16 abuts against the sheet-like therapeutic substance A at a certain point in time, thus restricting the pressing portion 15 from being compressed any further.

As shown in Fig. 4, in order to paste the sheet-like therapeutic substance A onto the pasting surface 2a of the sheet supporting portion 2 by using the thus-configured sheet pasting jig 10 according to this embodiment, the sheet supporting portion 2 is disposed with the pasting surface 2a facing vertically upward, the sheet-like therapeutic substance A having a greater area than the pasting surface 2a is mounted on the pasting surface 2a, and the blade portion 3 is lowered from thereabove.

At this time, because the pressing portion 15 is disposed inside the blade portion 3 at the position in which it protrudes further outward than the blade tip 3a, the pressing portion 15 comes into contact with the sheet-like therapeutic substance A before the blade portion 3 cuts the sheet-like therapeutic substance A. When the blade portion 3 is lowered further, the pressing portion 15 is compressed by being elastically deformed, thus bringing the blade tip 3a of the blade portion 3 into contact with the sheet-like therapeutic substance A. Thus, by lowering the blade portion 3 further from this state, as shown in Fig. 5, the therapeutic substance A is cut by the blade tip 3a.

In this case, with the sheet pasting jig 10 according to this embodiment, there is an advantage in that, because the pressing portion 15 continues to hold down the sheet-like therapeutic substance A against the pasting surface 2a of the sheet supporting portion 2 before cutting and also during cutting, the position of the therapeutic substance A does not shift and it is possible to stably perform the cutting task.

In addition, by providing the restricting member 16, it is possible to make the restricting member 16 abut against the therapeutic substance A before the pressing portion 15, which holds down the sheet-like therapeutic substance A, becomes so compressed that the therapeutic substance A is pressed with an excessively large force, and thus, even in the case in which the sheet-like therapeutic substance A is soft, the integrity thereof can be maintained.

In addition, as shown in Figs. 6 and 7, when cutting the sheet-like therapeutic substance A in such a manner that the blade tip 3a of the blade portion 3 abuts against the pasting surface 2a of the sheet supporting portion 2, because the blade portion 3 itself serves as a restricting member, it is permissible not to provide a separate restricting member.

In this case, it is preferable to provide a contact-pressure limiting mechanism that adjusts the contact pressure between the blade tip 3a and the pasting surface 2a so as to fall within a range greater than the pressure required to cut the therapeutic substance A and lower than the pressure that causes the pasting surface 2a to deform. The contact-pressure limiting mechanism is formed of, for example, a buffer member 17, such as a spring, that is provided between the plate member 13 and the pressing portion 15, as shown in Fig. 8. When the pressing portion 15 that has come into contact with the pasting surface 2a via the therapeutic substance A receives a greater pressing force than a predetermined level from the pasting surface 2a, the buffer member 17 is elastically deformed, thus absorbing the excessive pressing force. By providing such a contact-pressure limiting mechanism, the therapeutic substance A can be cut without damaging the pasting surface 2a with the blade tip 3a.

In addition, in the case in which the pressing portion 15 is provided, as shown in Fig. 9, it is preferable that the contact surface 15a of the pressing portion 15 that comes into contact with the therapeutic substance A be formed in a mesh pattern so as to serve as a sheet removing means for removing the therapeutic substance A that is punched out and remains inside the blade tip 3a from the blade tip 3a. For example, in the case in which the pressing portion 15 is formed of sponge, a coarse sponge may be used. In the case in which the pressing portion 15 is formed of rubber or the like, a plate having a mesh pattern may be pasted onto the contact surface 15a.

By forming the contact surface 15a of the pressing portion 15 in a mesh pattern, the contact area between the pasting surface 2a and the therapeutic substance A becomes larger than the contact area between the pressing portion 15 and the therapeutic substance A in the circular region cut out by the blade portion 3. Therefore, when the pressing portion 15 is raised after the therapeutic substance A is cut by the blade tip 3a, it is possible to make the circular therapeutic substance A easily peel off from the pressing portion 15 and to reliably leave the therapeutic substance A on the pasting surface 2a.

In addition, as shown in Figs. 10 to 12, in this embodiment, a sheet supporting portion 2 provided with a fold-back portion 2d that protrudes from the pasting surface 2a may be used. The fold-back portion 2d is provided in a ring shape slightly inside a line that comes into contact with the blade tip 3a so as to be parallel to this contact line. In addition, the fold-back portion 2d is formed in a hook shape which is bent inward.

As shown in Fig. 11, after placing the sheet-like therapeutic substance A on the fold-back portion 2d in the state in which the sheet-like therapeutic substance A is separated from the pasting surface 2a, the therapeutic substance A is cut in the same manner as shown in Figs. 6 and 7. At this time, as shown in Fig. 12, the circular therapeutic substance A that has been cut out with the blade portion 3 is pushed down onto the pasting surface 2a by the pressing portion 15, and is placed inside the ring-shaped fold-back portion 2d, as shown in Fig. 12. In this state, the inwardly bent fold-back portion 2d is hooked on a peripheral edge portion of the therapeutic substance A, thus preventing the therapeutic substance A from falling off the pasting surface 2a.

In addition, in this embodiment, as shown in Figs. 13 and 14, a liquid accommodating pack (liquid applying mechanism) 18a that accommodates liquid B, such as water or liquid adhesive, may be provided on the same side of the pressing portion 15 as the blade base. Furthermore, a needle (liquid applying mechanism) 18b that is secured to the pressing portion 15 with the needle point thereof pointing toward the liquid pack 18a is provided between the pressing portion 15 and the liquid pack 18a, and the pressing portion 15 is supported so as to be slightly movable in the direction toward the blade base of the blade portion 3.

As shown in Fig. 14, when the pressing portion 15 receives a pressing force from the pasting surface 2a via the therapeutic substance A, the pressing portion 15 is slightly raised, which causes the needle 18b to pierce the liquid pack 18a, and thus, the liquid B flows out of the liquid pack 18a. Subsequently, the pressing portion 15 is compressed, thus lowering the blade tip 3a to the pasting surface 2a. The liquid B that has flowed out of the liquid pack 18a passes through the pressing portion 15 formed of a sponge and infiltrates the therapeutic substance A. By applying the liquid B to the sheet-like therapeutic substance A in this way, the force with which the therapeutic substance A is adhered to the affected area is enhanced, and it is possible to facilitate the task of pasting the sheet-like therapeutic substance A onto the affected area.

### {Third Embodiment}

Next, a sheet pasting jig 20 according to a third embodiment of the present invention will be described below with reference to the drawings.

In describing this embodiment, the same reference signs are assigned to the parts having common configurations with those of the sheet pasting jig 10 according to the second embodiment described above, and descriptions thereof will be omitted.

As shown in Figs. 15 to 22, the sheet pasting jig 20 according to this embodiment differs from the sheet pasting jig 10 according to the second embodiment in that a ring-shaped blade portion 3 that cuts the sheet-like therapeutic substance A to be pasted onto a circular area, which is smaller than the sheet supporting portion 2, is included and that a base portion 21 on which the sheet supporting portion 2 is placed is included.

As shown in Fig. 16, the sheet pasting jig 20 according to this embodiment has a unit 22 including the bracket 12, the blade portion 3, the plate member 13, and the spring 14, which are similar to those of the sheet pasting jig 10 according to the second embodiment, and a base portion 21 that is attached to the unit 22 in a manner that allows the unit 22 to be separated therefrom. The base portion 21 is provided with a placement portion 23 on which the sheet supporting portion 2 is placed and a unit attaching portion 24 that secures the unit 22.

The placement portion 23 is provided with a placement surface 23a on which a back surface of the sheet supporting portion 2, which is on the opposite side of the pasting surface 2a thereof, is placed, and butting portions 23b and 23c that abut against two side surfaces that are perpendicular to each other, as shown in Fig. 17. The unit attaching portion 24 is provided with two depressions 24a so that protrusions (not shown) on the unit 22 side are fitted thereto. The protrusions on the unit 22 side, the depressions 24a, and the placement portion 23 constitute positioning portions with which the blade portion 3 is positioned with respect to the sheet supporting portion 2.

The operation of the thus-configured sheet pasting jig 20 according to this embodiment will be described below.

In order to paste the sheet-like therapeutic substance A onto the pasting surface 2a of the sheet supporting portion 2 by using the sheet pasting jig 20 according to this embodiment, the sheet supporting portion 2 whose pasting surface 2a is made to face vertically upward is placed on the base portion 21, in which the placement surface 23a of the placement portion 23 is disposed so as to face vertically upward, so that the back surface of the sheet supporting portion 2 comes into tight contact with the placement surface 23a, as shown in Fig. 17.

Then, the sheet supporting portion 2 is moved onto the placement surface 23a, and the two side surfaces of the sheet supporting portion 2 that are perpendicular to each other are made to individually abut against the butting portions 23b and 23c. By doing so, the sheet supporting portion 2 is positioned in the vertical direction and the horizontal direction with respect to the base portion 21, with the pasting surface 2a facing horizontally upward.

Next, as shown in Fig. 18, the sheet-like therapeutic substance A is placed on the pasting surface 2a of the sheet supporting portion 2. At this time, it is not necessary to precisely position the therapeutic substance A with respect to the pasting surface 2a, and it suffices to roughly dispose it so as to encompass a region onto which the therapeutic substance A is to be pasted.

In this state, as shown in Fig. 19, the unit 22 is attached by fitting the protrusions into the depressions 24a of the unit attaching portion 24 of the base portion 21. By doing so, the unit 22 is secured to the base portion 21 in a position-aligned state. Therefore, the blade portion 3 provided in the unit 22 is attached, in a position-aligned state, to the sheet supporting portion 2 mounted on the base portion 21 in a position-aligned state.

Next, as shown in Fig. 20, the plate member 13 and the blade portion 3 provided in the unit 22 are pushed downward against the biasing force of the spring 14. By doing so, the sheet-like therapeutic substance A is cut in a circular shape between the blade tip 3a of the blade portion 3 facing downward and the pasting surface 2a of the sheet supporting portion 2.

From this state, the unit 22 is removed from the base portion 21, as shown in Fig. 21, and the sheet-like therapeutic substance A is peeled off from the periphery, as shown in Fig. 22. By doing so, the sheet-like therapeutic substance A that has been cut out in a circular shape is left in the state in which the sheet-like therapeutic substance A is pasted onto the pasting surface 2a of the sheet supporting portion 2.

Here, the base portion 21 and the unit 22 are formed of separate components that can be separated from each other and can be assembled with the positions thereof set; however, alternatively, these two components may be provided in a state in which they are secured together in advance. In this case, the sheet supporting portion 2 on which the sheet-like therapeutic substance A is mounted should be inserted into a gap between the placement surface 23a of the base portion 21 and the blade tip 3a of the blade portion 3, and the two side surfaces of the sheet supporting portion 2 should be made to abut against the butting portions 23b and 23c.

Note that, in this embodiment, a scale 23d may be provided along the butting portion 23c, as shown in Fig. 23. The scale 23d may be provided along the butting portion 23b. By moving the sheet supporting portion 2 onto the placement surface 23a in the state in which the sheet supporting portion 2 abuts against the butting portion 23c, and by positioning the sheet supporting portion 2 with reference to the scale 23d, it is possible to adjust the position at which the therapeutic substance A is pasted onto the pasting surface 2a.

In addition, in this embodiment, as shown in Fig. 24, a base-portion protrusion 23e that is fitted into a supporting-portion depression 2e formed on the back surface of the sheet supporting portion 2 may be provided in the placement surface 23a. By fitting the base-portion protrusion 23e into the supporting-portion depression 2e also, it is possible to position the sheet supporting portion 2 with respect to the base portion 21. In this case, the butting portions 23b and 23c may be omitted.

In addition, in this embodiment, as shown in Fig. 25, the plate member 13 may be attached to the blade portion 3 in a removable manner so as to serve as a sheet removing means for removing the therapeutic substance A that is punched out and remains inside the blade tip 3a from the blade tip 3a. By removing the plate member 13 from the blade portion 3 after the therapeutic substance A is cut by the blade tip 3a, it is possible to hold down the therapeutic substance A against the pasting surface 2a by using an instrument such as forceps or the like inserted into the interior of the blade portion 3 so that the circular therapeutic substance A that has been cut out does not peel off from the pasting surface 2a.

In addition, in this embodiment, multiple types of units 22 having blade portions 3 whose diameters differ from each other may be provided, and the units 22 to be attached to the base portion 21 may be exchangeable. By exchanging the units 22 to be attached to the base portion 21, it is possible to change the size of the therapeutic substance A to be cut out.

In addition, in this embodiment, although two side surfaces of the sheet supporting portion 2 are made to abut against the butting portions 23b and 23c, alternatively, a fitting depression 25 having a shape complementary to the external shape of the sheet supporting portion 2 may be provided in the base portion 21, as shown in Figs. 26 and 27, and the sheet supporting portion 2 may be attached to the base portion 21 with the position thereof set by fitting the sheet supporting portion 2 into the fitting depression 25.

In addition, in the case in which the sheet-like therapeutic substance A is pasted over the entire pasting surface 2a of the sheet supporting portion 2, for example, as shown in Figs. 28 to 33, a fitting depression 25 having substantially the same shape as the pasting surface 2a of the sheet supporting portion 2 should be provided in the base portion 21, and, additionally, a blade portion 3 having a substantially equivalent shape to that of the fitting depression 25 should be used. The blade portion 3 is provided with a notch 3b that avoids a portion of the sheet supporting portion 2 at which a grip 2c is provided.

As shown in Fig. 33, a minute gap is formed at the fitting depression 25 of the base portion 21 in the area surrounding the sheet supporting portion 2 in the state in which the sheet supporting portion 2 is fitted thereto, and, when the blade portion 3 being lowered thereto is inserted into this minute gap, the sheet-like therapeutic substance A is cut by means of shearing between the blade portion 3 and the sheet supporting portion 2.

In this case also, from the state shown in Fig. 29, in which the unit 22 shown in Fig. 28 is removed from the base portion 21, the sheet supporting portion 2 is fitted into the fitting depression 25, as shown in Fig. 30, and the sheet-like therapeutic substance A is disposed so as to bridge the unit attaching portion 24 of the base portion 21 and the pasting surface 2a of the sheet supporting portion 2, as shown in Fig. 31. In this state, the unit 22 is attached to the unit attaching portion 24 with the position thereof set, and, by pushing down the blade portion 3, as shown in Fig. 32, it is possible to cut the therapeutic substance A so as to cover the entire pasting surface 2a of the sheet supporting portion 2 and also to paste the therapeutic substance A onto the pasting surface 2a.

Note that, in this embodiment, instead of the blade tip 3a extending in the direction perpendicular to the direction C in which the blade portion 3 is moved, as shown in Fig. 33, a blade tip 3a that is inclined with respect to the direction C in which the blade portion 3 is moved may be employed, as shown in Fig. 34. By doing so, when the blade portion 3 is moved vertically downward, because the blade tip 3a of the blade portion 3 gradually comes into contact with the therapeutic substance A instead of doing so all at once, a state in which the blade portion 3 and the therapeutic substance A are moved relative to each other in the direction parallel to the blade tip 3a is achieved, that is, the cut is made in a pulling manner. As a result, there is an advantage in that a smooth cut can be made even in the case in which the therapeutic substance A is soft.

It is also possible to obtain similar effects by forming the blade tip 3a in a corrugated shape, as shown in Fig. 35.

In addition, it is also possible to achieve similar effects by cutting the therapeutic substance A while rotating a cylindrically-shaped blade portion 3 about a center axis thereof, as shown in Fig. 36.

In the example shown in Fig. 36, a male screw 3c provided in the blade portion 3 is engaged with the female screw 12a provided in the bracket 12, and thus, by twisting the handle 3d provided in the blade portion 3, the blade portion 3 is lowered while rotating about the center axis thereof due to the coupling between the screws. By doing so, because the therapeutic substance A is cut while the blade portion 3 is moving in the circumferential direction along the direction parallel to the blade tip 3a, a smooth cut can be made in a pulling manner.

Note that, instead of the male screw 3c, a protrusion (not shown) that can be moved in a spiral-shaped groove (not shown) provided in the bracket 12 may be provided in the blade portion 3. Also, a protrusion that can be moved in a spiral-shaped groove provided in the blade portion 3 may be provided in the bracket 12.

In the configuration shown in Fig. 36, it is preferable to provide a blade-portion stopping mechanism that stops the lowering of the blade portion 3 at a position at which the blade tip 3a comes into contact with the pasting surface 2a. As shown in Fig. 37, the blade-portion stopping mechanism has a configuration in which, for example, a height dimension of the blade portion 3 and a height dimension of the bracket 12 are the same, and, when the blade tip 3a comes into contact with the pasting surface 2a, the top-end surface of the bracket 12 and the bottom-end surface of the handle 3d abut against each other in the top-to-bottom direction. By doing so, it is possible to prevent the blade tip 3a from wedging into the pasting surface 2a.

In addition, as shown in Fig. 38, a pressure applying means 26 for applying pressure to the ring-shaped blade portion 3 on the inside thereof may be provided. Examples of the pressure applying means 26 include a pipet provided with a bellows 26a, as shown in Fig. 38. The reference sign 27 in the figure indicates a notch provided in the handle 3d. Pressure is applied by pressing down a head portion of the bellows 26a by placing a finger in the notch 27, thus increasing the pressure in the internal space between the handle 3d and the blade tip 3a, which makes it possible to push out the therapeutic substance A that has entered inside the blade tip 3a.

Note that, in the case in which an air permeable component is employed as the sheet supporting portion 2, the sheet supporting portion 2 may be sucked from the back surface thereof to make the sheet-like therapeutic substance A adhere to the pasting surface 2a.

By doing so, it is possible to prevent the sheet-like therapeutic substance A that has been punched out by the ring-shaped blade portion 3 from remaining on the blade portion and being pulled off the pasting surface 2a together with the blade portion 3 when pulling up the blade portion 3.

In the configuration shown in Fig. 38, a film (liquid applying mechanism) 18c that closes the interior of the blade portion 3 may be provided, as shown in Fig. 39, and the liquid B, such as water or liquid adhesive, may be retained in the space above the film 18c. The strength that the film 18c possesses is such that it is torn open when pressure is applied by means of the bellows 26a. When pushing out the therapeutic substance A that has entered inside the blade tip 3a by pushing down the bellows 26a, the film 18c is torn open and the liquid B is applied to the therapeutic substance A. By doing so, the force with which the therapeutic substance A adheres to the affected area is enhanced, and it is possible to facilitate the task of pasting the therapeutic substance A onto the affected area.

In this case, it is preferable to provide, at a position closer to the blade tip 3a than to the film 18c, a liquid-sprinkling plate 18d in which numerous through-holes are formed in a uniform density so that the liquid B is uniformly applied to the therapeutic substance A. Furthermore, in order to prevent the liquid B from splashing, it is preferable that the base portion 21 be provided with a wall portion 18e that surrounds the fitting depression 25, as shown in Fig. 40. In addition, a liquid-absorbing member 18f that absorbs the liquid B may be provided inside the wall portion 18e.

In addition, the bellows 26a may be used simply as a tool for applying the liquid B to the therapeutic substance A instead of the pressure applying means 26. Specifically, as shown in Fig. 41, a pipet 29 that has the bellows 26a and that accommodates the liquid B may be provided in the internal space between the handle 3d and the blade tip 3a. It is preferable that the pipet 29 be provided with a nozzle 29a that sprays the liquid B in the form of mist. By pushing down the bellows 26a, it is possible to uniformly spray the liquid onto the therapeutic substance A from the nozzle 29a in the form of mist.

In addition, the blade portion 3 for cutting out a circular therapeutic substance A is not limited to those having a ring shape, and, as shown in Fig. 42, it is permissible to employ one provided with one or more blade portions 3 that can be moved closer to and away from the pasting surface 2a of the sheet supporting portion 2 and a rotating mechanism 28 that rotates the blade portions 3, which are separated in the horizontal direction, about a rotation shaft 28a. Fig. 42 shows an example having two blade portions 3 that can be disposed at positions away from the rotation shaft 28a at equal distances. It is possible to arbitrarily adjust the distances from the rotation shaft 28a, and it is possible to arbitrarily adjust the size of the therapeutic substance A to be pasted.

With such a sheet pasting jig also, the blade portions 3 can be moved in the direction parallel to the blade tips 3a with respect to the sheet-like therapeutic substance A, and thus, there is an advantage in that, due to the effect of making the cut in a pulling manner, a smooth cut can be made even with a soft jelly-like therapeutic substance A.

In the configuration shown in Fig. 42, it is permissible to provide a cutting-dimension adjusting mechanism that adjusts the distances between the two blade portions 3 and the rotation shaft 28a while keeping the distances between the two blade portions 3 and the rotation shaft 28a equal. The cutting-dimension adjusting mechanism is formed of, for example, two pinions 28c provided in an arm 28b and racks 28d integrally provided in the individual blade portions 3, as shown in Fig. 43. The arm 28b is secured to the rotation shaft 28a so as to perpendicularly intersect and support the blade portions 3 on both sides thereof. The pinions 28c are provided on both sides of the rotation shaft 28a. The racks 28d are disposed along the longitudinal direction of the arm 28b, and are engaged with the pinions 28c.

The two pinions 28c are rotated in synchronization at the same speed in the opposite directions from each other by means of a belt 28e bridging the two pinions 28c. When a user moves one of nobs 28f secured to the blade portions 3 and the racks 28d along the arm 28b, a linear motion of one of the racks 28d is transmitted to the other rack 28d as a linear motion in the opposite direction via the rotational motions of the pair of the pinions 28c. As a result, the two blade portions 3 are simultaneously moved by the same distances in the opposite directions from each other along the arm 28b. The arm 28b may be provided with scales (not shown) that indicate the distances between the blade portions 3 and the rotation shaft 28a.

In addition, as shown in Fig. 44, as another example of the cutting-dimension adjusting mechanism, a single blade portion 3 may be supported about a pivoting shaft 28g that is perpendicular to the rotation shaft 28a in a pivotable manner and in a manner in which the angle with respect to the rotation shaft 28a can be changed. By pivoting the blade portion 3 about the pivoting shaft 28g also, it is possible to arbitrarily adjust the diameter of the therapeutic substance A that is cut by the blade tip 3a rotated about the rotation shaft 28a. In this case, the blade portion 3 is provided in a manner in which the angle between the longitudinal direction thereof and the rotation shaft 28a can be temporarily fixed.

In addition, in the above-described embodiment, the therapeutic substance A has been described as an example of the sheet-like substance, and the affected area has been described as an example of a target site onto which the sheet-like substance A is pasted. Examples of the sheet-like therapeutic substance A include a hemostat such as TachoComb (made by CSL Behring) or the like, an anti-adhesive agent such as Seprafilm (made by Kaken Pharmaceutical Co. Ltd.) or the like, other types of adhesives, biological absorptive sheets that are absorbed in biological tissue, and so forth. In addition, a substance other than the therapeutic substance A may be employed as the sheet-like substance.

In addition, in this embodiment, the sheet supporting portion 2 may be provided in a form in which the sheet supporting portion 2 is held in the base portion 21 in a position-aligned state, as shown in Fig. 45. By doing so, trouble associated with installing the sheet supporting portion 2 in the base portion 21 is eliminated. A sheet pasting jig 30, which is formed of the integrally assembled base portion 21, the unit 22, and the sheet supporting portion 2, is provided in a state in which the entirety thereof is sterilized and is exchanged with a new sheet pasting jig 30 each time it is used. The sheet-like therapeutic substance A is inserted between the pasting surface 2a and the blade tip 3a, thus being placed on the pasting surface 2a.

Although it is permissible that the base portion 21 and the unit 22 can be separated from each other, these two components may be secured to each other. In the case in which the base portion 21 and the unit 22 are secured to each other, the fitting depression 25 is formed so that the dimension thereof corresponding to the width direction of the sheet supporting portion 2 is uniform, so that the sheet supporting portion 2 can be pulled out of the fitting depression 25 by moving the sheet supporting portion 2 in the longitudinal direction of the grip 2c. The reference sign 19 indicates holding portions that hold down peripheral edge portions of the pasting surface 2a of the sheet supporting portion 2 in the fitting depression 25.

After cutting the therapeutic substance A in a similar manner to those shown in Figs. 19 and 20, the sheet supporting portion 2 is pulled toward the base side of the grip 2c and removed from the base portion 21 via the fitting depression 25, as indicated by two-dot chain lines in Fig. 45, and thus, the circular sheet-like therapeutic substance A that has been cut out is left in the state in which the circular sheet-like therapeutic substance A is pasted onto the pasting surface 2a of the sheet supporting portion 2.

In the configuration shown in Fig. 45, a stage 31 that can be moved in the depth direction of the fitting depression 25 may be provided in the fitting depression 25 and the sheet supporting portion 2 may be placed on this stage 31, as shown in Figs. 46 to 48. An elastic member 32 that biases the stage 31 toward the blade tip 3a is provided between the stage 31 and the placement surface 23a, and, when the elastic member 32 contracts due to a pressing force from the blade portion 3, as shown in Fig. 47, the sheet supporting portion 2 is lowered in the fitting depression 25.

Furthermore, protrusions 33 formed of an elastic material are provided at side surfaces of the sheet supporting portion 2, and fitting grooves 34, into which the protrusions 33 are fitted, are formed in the butting portion 23b of the fitting depression 25. The fitting grooves 34 are formed over the entire length in the direction in which the sheet supporting portion 2 is pulled out and serve as guide rails in which the protrusions are moved when pulling out the sheet supporting portion 2 from the fitting depression 25.

As shown in Fig. 48, the stage 31 and the sheet supporting portion 2, which are lowered in the fitting depression 25, are stopped when the protrusions 33 are fitted into the fitting grooves 34 when the positions of the protrusions 33 are aligned with the positions of the fitting grooves 34. In this state, the circular therapeutic substance A that has been cut out is positioned on the pasting surface 2a, and the rest of the therapeutic substance A is left on the top surface of the unit attaching portion 24. Therefore, by pulling out the sheet supporting portion 2 from the fitting depression 25, it is possible to easily remove the sheet supporting portion 2 from the base portion 21.

Note that, in the first to third embodiments described above, it is permissible to use a sheet supporting portion 2 that is provided with, as shown in Fig. 49, a marker 2f on the back surface thereof at a position corresponding to a position on the pasting surface 2a at which the therapeutic substance A is cut by the blade tip 3a, that is, a position at which the therapeutic substance A is pasted. When pasting the therapeutic substance A onto an affected area, the user cannot visually check the pasting surface 2a. In such a situation also, the position of the therapeutic substance A can be ascertained based on the marker 2f on the back surface, and therefore, it is possible to easily position the therapeutic substance A at the position at which it is to be pasted onto the affected area. The same effect can be obtained by forming the sheet supporting portion 2 by using a transparent member.

### {Reference Signs List}

A therapeutic substance (substance)
1, 10, 20 sheet pasting jig
2 sheet supporting portion
2a pasting surface
2d fold-back portion
2e supporting-portion depression
2f marker
3 blade portion
3a blade tip
3c male screw (moving mechanism)
4 protrusion (positioning portion)
11 fitting portion
12a female screw (moving mechanism)
13 plate member
15 pressing portion
16 restricting member
17 buffer member (contact-pressure limiting mechanism)
23a, 23b butting portion
23e base-portion protrusion
25 fitting depression
26 pressure applying means (removing means)
28 rotating mechanism (moving mechanism)

## Claims

1. A sheet pasting jig comprising:
a blade portion that is brought into close contact with a pasting surface, which is a surface of a sheet supporting portion onto which a sheet-like substance is pasted, and that is capable of cutting the sheet-like substance between the sheet supporting portion and the blade portion into a piece having an area equivalent to or less than that of the pasting surface; and
a positioning portion that positions the blade portion and the sheet supporting portion.

2. The sheet pasting jig according to Claim 1, wherein the positioning portion has a butting portion that is made to abut against two or more surfaces that intersect each other and that are surfaces other than the pasting surface of the sheet supporting portion.

3. The sheet pasting jig according to Claim 2,
wherein the sheet supporting portion is formed in a flat plate shape, and
the butting portion abuts against a back surface and two adjacent side surfaces of the pasting surface of the sheet supporting portion.

4. The sheet pasting jig according to any one of Claims 1 to 3, wherein the positioning portion has a fitting portion that is fitted to the sheet supporting portion.

5. The sheet pasting jig according to Claim 4, wherein the fitting portion is formed in a depressed shape or a protruding shape that is fitted to a protrusion or a depression provided in the sheet supporting portion.

6. The sheet pasting jig according to Claim 4 or 5, wherein the fitting portion has a fitting depression having a shape complementary to an external shape of the sheet supporting portion so that an outer surface of the sheet supporting portion is fitted thereto.

7. The sheet pasting jig according to any one of Claims 2 to 6, further comprising:
a base portion that has a placement portion on which the sheet supporting portion is placed and to which the blade portion is attached,
wherein the positioning portion has a base-portion protrusion that is provided in the placement portion and that is fitted to a supporting-portion depression that is formed in a surface on the opposite side of the pasting surface of the sheet supporting portion.

8. The sheet pasting jig according to any one of Claims 1 to 6, wherein, when a blade tip of the blade portion cuts the sheet-like substance, the blade portion and the sheet-like substance are moved relative to each other in a direction parallel to the blade tip.

9. The sheet pasting jig according to Claim 7, wherein a blade tip of the blade portion is inclined with respect to a direction in which the blade portion is moved.

10. The sheet pasting jig according to Claim 7, further comprising:
a moving mechanism that moves a blade tip of the blade portion in a direction parallel to the blade tip relative to the sheet-like substance.

11. The sheet pasting jig according to Claim 9, wherein the blade tip is formed in a corrugated shape along the direction in which the blade portion is moved.

12. The sheet pasting jig according to Claim 9 or 10,
wherein the blade tip of the blade portion is formed in a ring shape, and
the moving mechanism brings the blade portion into close contact with the sheet-like substance while rotating the blade portion about a center axis of the blade tip.

13. The sheet pasting jig according to any one of Claims 8 to 12,
wherein the blade tip of the blade portion is formed in a ring shape, the sheet pasting jig further comprising:
a sheet removing means for removing the sheet-like substance, which has been punched out and remains inside the blade tip, from the blade tip.

14. The sheet pasting jig according to Claim 13, wherein the sheet removing means is provided with a plate member that is attached to the blade portion in a removable manner and that can entirely cover the surface of the sheet supporting portion.

15. The sheet pasting jig according to Claim 13, further comprising:
a pressing portion that is formed of an elastic material that can be elastically deformed between a position at which the elastic material protrudes further outward than the blade tip of the blade portion and a position at which the elastic material is retracted further inward than the blade tip;
wherein the sheet removing means is configured by forming a contact surface of the pressing portion, which comes into contact with the sheet-like substance on the pasting surface, in a mesh pattern.

16. The sheet pasting jig according to any one of Claims 1 to 12, wherein the blade portion is secured to the plate member, which can entirely cover the surface of the sheet supporting portion.

17. The sheet pasting jig according to any one of Claims 1 to 13, further comprising:
a pressing portion that is formed of an elastic material that can be elastically deformed between a position at which the elastic material protrudes further outward than the blade tip of the blade portion and a position at which the elastic material is retracted further inward than the blade tip.

18. The sheet pasting jig according to Claim 14, further comprising:
a restricting member that is disposed at a position at which the restricting member is retracted further inward than the blade tip of the blade portion and that restricts the pressing portion from elastically deforming any further.

19. A sheet pasting jig further comprising:
the sheet supporting portion that is held by means of the positioning portion in a position-aligned state with respect to the blade portion.

20. The sheet pasting jig according to Claim 19, wherein, on a back surface on the opposite side of the pasting surface, the sheet supporting portion has a marker that indicates a position at which the sheet-like substance is to be cut by the blade portion.

21. The sheet pasting jig according to Claim 20 or 21, wherein, at the pasting surface, the sheet supporting portion has a fold-back portion that protrudes from the pasting surface in the vicinity of the position at which the sheet-like member is to be cut by the blade portion and that has a hook shape that is bent inward of a region to be cut by the blade portion.

22. The sheet pasting jig according to any one of Claims 1 to 21, further comprising:
a cutting-dimension adjusting mechanism that can change a dimension of the sheet-like substance to be cut by the blade portion.

23. The sheet pasting jig according to Claim 22, further comprising: a rotating mechanism that supports the blade portion in a rotatable manner about a predetermined rotation axis,
wherein the cutting-dimension adjusting mechanism changes a distance between the blade portion and the predetermined rotation axis.

24. The sheet pasting jig according to Claim 22, further comprising:
a rotating mechanism that supports the blade portion in a rotatable manner about a predetermined rotation axis,
wherein the cutting-dimension adjusting mechanism changes an angle of the blade portion with respect to the predetermined rotation axis.

25. The sheet pasting jig according to Claim 22, wherein the cutting-dimension adjusting mechanism is provided with a plurality of the blade portions that have blade tips whose dimensions differ from each other and that are exchangeable.

26. The sheet pasting jig according to any one of Claims 1 to 25, further comprising:
a blade-portion stopping mechanism that stops the movement of the blade portion in the direction in which the blade portion approaches the pasting surface at a position at which the blade portion comes into contact with the pasting surface.

27. The sheet pasting jig according to any one of Claims 1 to 26, further comprising:
a contact-pressure limiting mechanism that limits a contact pressure of the blade portion against the pasting surface within a range that is greater than a pressure required to cut the sheet-like member and that is lower than a pressure that causes the pasting surface to deform.

28. The sheet pasting jig according to any one of Claims 1 to 27, further comprising:
a liquid applying mechanism that applies liquid to the pasting surface.

29. A pasting method for a sheet-like substance employing the sheet pasting jig according to any one of Claims 1 to 28, comprising:
a disposing step of disposing the sheet-like substance on the surface of the sheet supporting portion;
a positioning step of positioning the sheet supporting portion and the sheet pasting jig; and
a cutting step of cutting the sheet-like substance by means of the blade portion of the sheet pasting jig.

30. The sheet pasting method according to Claim 29, wherein the cutting step is performed while covering above the sheet-like substance disposed on the surface of the sheet supporting portion.

31. The sheet pasting method according to Claim 10 or 30, wherein the cutting step is performed while holding down the sheet-like substance, which is disposed on the surface of the sheet supporting portion, against the sheet supporting portion, before cutting and also during cutting.

32. The sheet pasting method according to any one of Claims 29 to 31, wherein the cutting step is performed while moving a blade tip of the blade portion and the sheet-like substance relative to each other in a direction parallel to the blade tip.

## Amended claims

### Amended claims under Art. 19.1 PCT

1. A sheet pasting jig comprising:
a blade portion that is brought into close contact with a pasting surface, which is a surface of a sheet supporting portion onto which a sheet-like substance is pasted, and that is capable of cutting the sheet-like substance between the sheet supporting portion and the blade portion into a piece having an area equivalent to or less than that of the pasting surface; and
a positioning portion that positions the blade portion and the sheet supporting portion.

2. The sheet pasting jig according to Claim 1, wherein the positioning portion has a butting portion that is made to abut against two or more surfaces that intersect each other and that are surfaces other than the pasting surface of the sheet supporting portion.

3. The sheet pasting jig according to Claim 2,
wherein the sheet supporting portion is formed in a flat plate shape, and
the butting portion abuts against a back surface and two adjacent side surfaces of the pasting surface of the sheet supporting portion.

4. The sheet pasting jig according to any one of Claims 1 to 3, wherein the positioning portion has a fitting portion that is fitted to the sheet supporting portion.

5. The sheet pasting jig according to Claim 4, wherein the fitting portion is formed in a depressed shape or a protruding shape that is fitted to a protrusion or a depression provided in the sheet supporting portion.

6. The sheet pasting jig according to Claim 4 or 5, wherein the fitting portion has a fitting depression having a shape complementary to an external shape of the sheet supporting portion so that an outer surface of the sheet supporting portion is fitted thereto.

7. The sheet pasting jig according to any one of Claims 2 to 6, further comprising:
a base portion that has a placement portion on which the sheet supporting portion is placed and to which the blade portion is attached,
wherein the positioning portion has a base-portion protrusion that is provided in the placement portion and that is fitted to a supporting-portion depression that is formed in a surface on the opposite side of the pasting surface of the sheet supporting portion.

8. The sheet pasting jig according to any one of Claims 1 to 6, wherein, when a blade tip of the blade portion cuts the sheet-like substance, the blade portion and the sheet-like substance are moved relative to each other in a direction parallel to the blade tip.

9. The sheet pasting jig according to Claim 7, wherein a blade tip of the blade portion is inclined with respect to a direction in which the blade portion is moved.

10. The sheet pasting jig according to Claim 7, further comprising:
a moving mechanism that moves a blade tip of the blade portion in a direction parallel to the blade tip relative to the sheet-like substance.

11. The sheet pasting jig according to Claim 9, wherein the blade tip is formed in a corrugated shape along the direction in which the blade portion is moved.

12. The sheet pasting jig according to Claim 9 or 10,
wherein the blade tip of the blade portion is formed in a ring shape, and
the moving mechanism brings the blade portion into close contact with the sheet-like substance while rotating the blade portion about a center axis of the blade tip.

13. The sheet pasting jig according to any one of Claims 8 to 12,
wherein the blade tip of the blade portion is formed in a ring shape, the sheet pasting jig further comprising:
a sheet removing means for removing the sheet-like substance, which has been punched out and remains inside the blade tip, from the blade tip.

14. The sheet pasting jig according to Claim 13, wherein the sheet removing means is provided with a plate member that is attached to the blade portion in a removable manner and that can entirely cover the surface of the sheet supporting portion.

15. The sheet pasting jig according to Claim 13, further comprising:
a pressing portion that is formed of an elastic material that can be elastically deformed between a position at which the elastic material protrudes further outward than the blade tip of the blade portion and a position at which the elastic material is retracted further inward than the blade tip;
wherein the sheet removing means is configured by forming a contact surface of the pressing portion, which comes into contact with the sheet-like substance on the pasting surface, in a mesh pattern.

16. The sheet pasting jig according to any one of Claims 1 to 12, wherein the blade portion is secured to the plate member, which can entirely cover the surface of the sheet supporting portion.

17. The sheet pasting jig according to any one of Claims 1 to 13, further comprising:
a pressing portion that is formed of an elastic material that can be elastically deformed between a position at which the elastic material protrudes further outward than the blade tip of the blade portion and a position at which the elastic material is retracted further inward than the blade tip.

18. The sheet pasting jig according to Claim 14, further comprising:
a restricting member that is disposed at a position at which the restricting member is retracted further inward than the blade tip of the blade portion and that restricts the pressing portion from elastically deforming any further.

19. The sheet pasting jig according to any one of Claims 1 to 18, further comprising:
the sheet supporting portion that is held by means of the positioning portion in a position-aligned state with respect to the blade portion.

20. The sheet pasting jig according to Claim 19, wherein, on a back surface on the opposite side of the pasting surface, the sheet supporting portion has a marker that indicates a position at which the sheet-like substance is to be cut by the blade portion.

21. The sheet pasting jig according to Claim 19 or 20, wherein, at the pasting surface, the sheet supporting portion has a fold-back portion that protrudes from the pasting surface in the vicinity of the position at which the sheet-like member is to be cut by the blade portion and that has a hook shape that is bent inward of a region to be cut by the blade portion.

22. The sheet pasting jig according to any one of Claims 1 to 21, further comprising:
a cutting-dimension adjusting mechanism that can change a dimension of the sheet-like substance to be cut by the blade portion.

23. The sheet pasting jig according to Claim 22, further comprising: a rotating mechanism that supports the blade portion in a rotatable manner about a predetermined rotation axis,
wherein the cutting-dimension adjusting mechanism changes a distance between the blade portion and the predetermined rotation axis.

24. The sheet pasting jig according to Claim 22, further comprising:
a rotating mechanism that supports the blade portion in a rotatable manner about a predetermined rotation axis,
wherein the cutting-dimension adjusting mechanism changes an angle of the blade portion with respect to the predetermined rotation axis.

25. The sheet pasting jig according to Claim 22, wherein the cutting-dimension adjusting mechanism is provided with a plurality of the blade portions that have blade tips whose dimensions differ from each other and that are exchangeable.

26. The sheet pasting jig according to any one of Claims 1 to 25, further comprising:
a blade-portion stopping mechanism that stops the movement of the blade portion in the direction in which the blade portion approaches the pasting surface at a position at which the blade portion comes into contact with the pasting surface.

27. The sheet pasting jig according to any one of Claims 1 to 26, further comprising:
a contact-pressure limiting mechanism that limits a contact pressure of the blade portion against the pasting surface within a range that is greater than a pressure required to cut the sheet-like member and that is lower than a pressure that causes the pasting surface to deform.

28. The sheet pasting jig according to any one of Claims 1 to 27, further comprising:
a liquid applying mechanism that applies liquid to the pasting surface.

29. A pasting method for a sheet-like substance employing the sheet pasting jig according to any one of Claims 1 to 28, comprising:
a disposing step of disposing the sheet-like substance on the surface of the sheet supporting portion;
a positioning step of positioning the sheet supporting portion and the sheet pasting jig; and
a cutting step of cutting the sheet-like substance by means of the blade portion of the sheet pasting jig.

30. The sheet pasting method according to Claim 29, wherein the cutting step is performed while covering above the sheet-like substance disposed on the surface of the sheet supporting portion.

31. The sheet pasting method according to Claim 10 or 30, wherein the cutting step is performed while holding down the sheet-like substance, which is disposed on the surface of the sheet supporting portion, against the sheet supporting portion, before cutting and also during cutting.

32. The sheet pasting method according to any one of Claims 29 to 31, wherein the cutting step is performed while moving a blade tip of the blade portion and the sheet-like substance relative to each other in a direction parallel to the blade tip.
